# EUROPEAN PATENT SPECIFICATION

(11) **EP 3 076 825 B1**
(45) Date of publication and mention of the grant of the patent: **07.11.2018**
(21) Application number: 14867259.5
(22) Date of filing: 03.12.2014
(51) Int. Cl.: A46B 15/00, A46B 13/02, A61C 17/22, A61N 5/06, A61C 19/06, A61C 17/16, A46B 9/04, A46B 5/00

(54) **SHORT WAVELENGTH VISIBLE LIGHT-EMITTING TOOTHBRUSH WITH AN ELECTRONIC SIGNAL INTERLOCK CONTROL**
SICHTBARES LICHT MIT KURZER WELLENLÄNGE EMITTIERENDE ZAHNBÜRSTE MIT VERRIEGELUNGSSTEUERUNG DURCH EIN ELEKTRONISCHES SIGNAL
BROSSE À DENTS ÉMETTRICE DE LUMIÈRE VISIBLE À LONGUEURS D'ONDES COURTES COMPRENANT UNE COMMANDE DE VERROUILLAGE À SIGNAL ÉLECTRONIQUE

(30) Priority: 05.12.2013 US 201361912260 P; 03.01.2014 US 201461923381 P; 21.08.2014 US 201414464895
(43) Date of publication of application: 12.10.2016
(73) Proprietor: Oralucent, LLC, Long Beach, CA 90803 (US)
(72) Inventor: DOOLEY, Thomas Jason, Hoboken, NJ 07030 (US); SHEPHERD, Greg, Fruitland Park, FL 34731 (US); LAWRENCE, Timothy, Lomg Beach, CA 90803 (US); BARNES, Mike, Dunnellon, FL 34432 (US); ZASTAWNY, Mathieu Julien Jean Emile, Jersey City, NJ 07302 (US)
(74) Representative: Ashton, Gareth Mark
(86) International application number: PCT/US2014/068379
(87) International publication number: WO 2015/084962

(56) References cited:
- EP-A1- 2 617 319
- WO-A1-2009/141489
- WO-A1-2011/077290
- US-A- 6 081 957
- US-A1- 2008 131 834
- US-A1- 2008 131 834
- US-A1- 2013 137 074
- US-B2- 6 902 397

## Description

### Background of the Invention

The present invention relates to increasing the safety of a dental hygiene implement such as a light-emitting manual or an electrically operated motorized toothbrush which emits radiation, in the violet and/or blue region of the visible spectrum, between 400nm and 500nm, in order to:
- oxidize and destroy potentially harmful bacteria and/or other contaminants or compounds contained within the mouth without harming or destroying human cells;
- exert a phototoxic effect on pathogenic periodontal and oral bacteria such as; P. Gingivalis and F. Nucleatum, and S. Mutans;
- activate a photo catalyst that may be deposited on the teeth and the gums of the person utilizing the toothbrush during normal brushing; and/or
- accelerate the whitening effects of a tooth bleaching agent added to toothpaste or toothgel such as carbamide peroxide or hydrogen peroxide.

The electronic interlock control mechanism in this toothbrush will reduce the possibility of accidental direct eye exposure to high flux visible light radiation emitted from this toothbrush when it is removed from the mouth.

Light-emitting toothbrushes have been developed over the past several years for teeth whitening applications in addition to the known oral hygiene benefits of regular brushing. When combined with a teeth whitening agent such as carbamide peroxide or hydrogen peroxide, studies have shown that light in the 400-500nm range accelerates the whitening effect of these agents. Wolfgang Buchallaa, Thomas Attina: External bleaching therapy with activation by heat, light or laser-A systematic review*;* Karen Luk, D.D.S.; Laura Tam, D.D.S., M.Sc.; Manfred Hubert, Ph.D.: Effect of light energy on peroxide tooth bleaching*.*

In addition, violet light in the 400nm-420nm range has been shown to have a phototoxic effect on pathogenic oral bacteria such as P. Gingivalis, S. Mutans and others. Michelle Maclean, Scott J. MacGregor, John G. Anderson, and Gerry Woolsey: Inactivation of Bacterial Pathogens following Exposure to Light from a 405-Nanometer Light-Emitting Diode Array*.* Doron Steinberg, Daniel Moreinos, John Featherstone, Moshe Shemesh, and Osnat Feuerstein: Genetic and Physiological Effects of Noncoherent Visible Light Combined with Hydrogen Peroxide on Streptococcus mutants in Biofilm*.* The inventors have previously shown the use of a light-emitting diode (LED) within a toothbrush provides anti-microbial properties of benefit to the oral hygiene of the end-user.

Current light-emitting toothbrushes have a manual on/off switch which activates the light-emitting device. This manual activation mechanism may lead to a safety risk because the user may activate the light and expose his or her eyes to high levels of light that may be harmful to the retina or optic nerve. The potentially harmful properties of visible light and maximum exposure levels are documented in ANSI standards. François C. Delori, Robert H. Webb, David H. Sliney: Maximum permissible exposures for ocular safety (ANSI 2000*), with emphasis on ophthalmic devices.* David H. Sliney, M.S.: Biohazards of Ultraviolet, Visible and Infrared Radiation*.* For example, the maximum permissible radiant power (thermal and photo-acoustic) entering a dilated pupil is 1.5 x 10⁻⁴ Watts. This limit would be exceeded if a user were to stare at a 420nm LED of 250mW radiant flux at a distance of 10cm for a period of 0.5 seconds. To prevent accidental eye exposure, a special electronic interlock control mechanism has been implemented to keep the optical source turned off if the toothbrush is not inserted in the user's mouth. The control mechanism will turn the optical source off immediately if the toothbrush is removed from the mouth prior to completing the brushing cycle.

A toothbrush is typically used in close proximity to the eyes of the user, and if ocular exposure to the light lasts several seconds, eye damage may occur. Furthermore, the ocular safety risk of manually activated light precludes the use of more powerful light-emitting devices such as high-powered LEDs, laser diodes, or vertical cavity surface emitting lasers, which would increase the teeth-whitening and antimicrobial benefits in proportion to the energy delivered. For example, studies show that effective whitening treatments require a minimum energy density of 30-50 J cm⁻² to produce noticeable shade whitening. However, such energy levels would not be readily achievable with a typical two minute brushing interval using a low-powered LED that would also be safe when directly placed in front of the eyes, even when used over a period of several weeks. Similar limitations exist for the anti-microbial properties of violet light as well.

It is therefore desirable to control the "on" state of the light-emitting device to a time period when it is in use in the oral cavity but to shut "off this high power light source immediately, when it is removed from the mouth to prevent direct eye exposure. This feature would also extend battery life of a battery operated brush since power is only used to illuminate the light source when in direct contact with the oral cavity. US 2008/0131834 discloses a toothbrush comprising a handle and a head at a distal end. The head has a blue light source and a sensor for measuring or detecting conductivity to determine when the head is in the mouth of a user.

### Summary of the Invention

This invention relates to increasing the safety of a dental hygiene implement that emits radiation in the violet and/or blue region of the visible spectrum, between 400nm and 500nm, by determining whether the implement is within the user's mouth or outside of the user's mouth. Direct eye exposure to such light can be avoided using a combination of sensors that deactivate the light source whenever it is outside the environment of the mouth and permits activation only when inside the mouth of the user. Specifically, the invention provides a toothbrush as defined in the appended claim 1. Other embodiments are not a part of the invention and are described for illustrative purposes only. The light emitting toothbrush, according to the present invention, includes a control circuitry which will typically be located in the handle and may include functions such as a timer circuitry (which times the duration(s) of use of the toothbrush while brushing), an on/off duty cycle of the violet light source or sources, a battery replacement indicator, and so on.
Preferably the driver, for driving the violet light source, is equipped with constant electrical current control electronics and a suitable driver is supplied by Linear Technology, of San Jose Calif., as part no. LTC3454 which is an integrated circuit high current LED driver. The control circuitry may also include violet light source control circuitry, which is connected with one or more sensors located in the brush head and the handle, for detecting when the brush head is actually located within a user's mouth, thereby reducing the possibility of the violet light being inadvertently emitted except when the toothbrush is actually located within the mouth of the user.

An AC or DC signal loop sensor establishes a signal loop through the user's mouth to the user's hand grasping the handle of the toothbrush to verify if the toothbrush head is within the user's mouth. If the sensor establishes a signal loop, the light source remains on until the toothbrush head is removed from the user's mouth breaking the signal loop. Once the signal loop is broken, the light source may be extinguished as soon as the brush head is removed from the mouth.

### Brief Description of the Drawings

The invention will be better understood by a reading of the Detailed Description of the Examples of the Invention along with a review of the drawings, in which:
Figure 1 shows the completion of a signal loop by a user inserting a toothbrush into his mouth;
Figure 2 depicts a representative oral care instrument, a toothbrush, illustrating various aspects;
Figure 3 depicts an alternate embodiment in which the wet bristles of the brush head are conductive to form a contact for completion of the signal loop;
Figure 4 shows a toothbrush with conductive metal pads;
Figure 5 shows a toothbrush with conductive plastic pads;
Figure 6 is a schematic of an exemplary control circuit;
Figure 7 is a schematic of an exemplary oral care instrument that has a replaceable head and neck;
Figure 8 is a schematic of an exemplary oral care instrument that has a replaceable brush head;
Figure 9 is an exploded view of a toothbrush with a capacitive sensor;
Figure 10 is a circuit view of the embodiment shown in Figure 9;
Figure 11 is a partial cross-sectional view of an embodiment where the sensor is a capacitive displacement sensor placed on the handle;
Figure 12 is a circuit view of an embodiment where the sensor is a capacitive displacement sensor;
Figure 13 is depicts an alternate embodiment where the sensor is an inductive proximity sensor;
Figure 14 shows an embodiment where the sensor is a passive thermal sensor placed near the upper portion of the handle of a toothbrush;
Figure 15 shows an embodiment where two passive thermal sensors are placed near the upper portion of the handle of a toothbrush;
Figure 16 shows an embodiment where the sensor is a passive thermal sensor placed on the back of the bristle plate of a toothbrush;
Figure 17 shows an embodiment where the sensor is an active thermal infrared sensor placed on the back of the bristle plate of a toothbrush;
Figure 18 shows an embodiment where the sensor is a passive optical sensor placed on the back of the bristle plate of a toothbrush;
Figure 19 shows an embodiment where the sensor is a photocell placed on the brush head of a toothbrush;
Figure 20 shows an embodiment where the sensor is an ultrasonic sensor placed on the brush head of a toothbrush;
Figure 21 is a partially exploded view of another embodiment where the sensor is a pressure sensing system;
Figure 22 is a side view of a portion of the pressure sensing system of Figure 21;
Figure 23 is a perspective view of the pressure sensing system of Figure 21;
Figure 24 is an exploded view of the pressure sensing system of Figure 21;
Figure 25 is a side elevational view of a portion of the pressure sensing system of Figure 21;
Figure 26 is a side elevational view of another portion of the pressure sensing system of Figure 21;
Figure 27 is a cross-sectional view of a portion of the pressure sensing system of Figure 21;
Figure 28 is a schematic drawing showing a circuit according to another embodiment where the sensor is a pressure sensing system;
Figure 29 is a schematic perspective view, in partial cross-section, of a toothbrush having a pressure sensing system according to another embodiment;
Figure 30 is an enlarged detail view of a portion of the toothbrush shown in Figure 29;
Figure 31 is an enlarged detail view, in partial cross-section, of a toothbrush head according to another embodiment where the sensor is a pressure sensing system;
Figure 32 is an enlarged detail view, in partial cross-section, of a toothbrush head according to another embodiment where the sensor is a pressure sensing system;
Figure 33 is an enlarged detail view, in partial cross-section, of a toothbrush head according to another embodiment where the sensor is a pressure sensing system;
Figure 34 is a detail view of the toothbrush head of Figure 33 taken along 507-507;
Figure 35 is a simplified descriptive view of another embodiment where the sensor is a pressure sensing system;
Figure 36 is a partial fragmentary view of a switch used in the toothbrush shown in Figure 35, the switch being shown in a first position;
Figure 37 is a partial fragmentary view of the switch shown in Figure 36, the switch being shown in a second position;
Figure 38 is a simplified wiring schematic for the toothbrush shown in Figure 35;
Figure 39 is a perspective view of another embodiment where the sensor is a pressure sensing system including a one-piece compressible portion;
Figure 40 is a perspective view of another embodiment where the sensor is a pressure sensing system including a two-piece compressible portion;
Figure 41 is a partial fragmentary perspective view of another embodiment;
Figure 42 is a detail view of a switch shown in Figure 41;
Figure 43 is a detail view of the switch shown in Figure 42, the switch being shown in a closed position;
Figure 44 is a partial fragmentary perspective view of another embodiment where the oral care instrument is a toothbrush having a Hall effect sensor;
Figure 45 is a partial fragmentary perspective view of a toothbrush in accordance with another embodiment;
Figure 46 is a partial fragmentary perspective view of another embodiment;
Figure 47 is a simplified wiring schematic illustrating an electrical circuit that can be used with the toothbrush shown in Figure 46;
Figure 48 is a partial fragmentary perspective view of another embodiment;
Figure 49 is a simplified wiring schematic illustrating a circuit that can be used with the toothbrush shown in Figure 48;
Figure 50 shows a simplified descriptive view of a toothbrush in accordance with another embodiment where the sensor is a pressure sensor;
Figure 51 shows a simple wiring schematic for the toothbrush shown in Figure 50;
Figure 52 shows a simple wiring schematic for a toothbrush that includes a three position switch;
Figure 53 shows a simple wiring schematic for a toothbrush with only one switch;
Figure 54 shows an exploded view of the toothbrush shown in Figure 50;
Figure 55 shows an alternative configuration for the contact plates shown in Figure 54;
Figure 56 shows the contact plates of Figure 55 when a force is applied to the bristle head of the toothbrush;
Figure 57 shows the contact plates of Figure 55 when the force applied to the bristle head exceeds a predetermined level;
Figure 58 shows a perspective view of a portion of a toothbrush in accordance with another embodiment where the sensor is a pressure sensor;
Figure 59 shows a partially exploded perspective view of a portion of the toothbrush shown in Figure 58;
Figure 60 shows a sectional view of a portion of the toothbrush shown in Figure 58;
Figure 61 shows another sectional view of the toothbrush shown in Figure 58;
Figure 62 shows a partially exploded perspective view of a portion of a toothbrush in accordance with another embodiment where the sensor is a pressure sensor;
Figure 63 shows a sectional view of a portion of the toothbrush shown in Figure 62;
Figure 64 shows a perspective view of a toothbrush having a one-piece compressible portion in the handle in accordance with another embodiment;
Figure 65 shows a perspective view of a toothbrush having a two-piece compressible portion in the handle in accordance with another embodiment;
Figure 66 shows a simplified descriptive view of a toothbrush in accordance with another embodiment where the sensor is a pressure sensor;
Figure 67 shows an enlarged detail of a switch shown in Figure 66, the switch being shown in a first position;
Figure 68 shows the switch from Figure 67 in a second position;
Figure 69 shows a simplified descriptive view of a toothbrush in accordance with another embodiment;
Figure 70 depicts a toothbrush illustrating various aspects of another embodiment wherein the sensor is a moisture sensor;
Figure 71 depicts another embodiment of a toothbrush wherein the sensor is a moisture sensor;
Figure 72 shows an embodiment wherein the sensor is an accelerometer; and
Figure 73 shows an embodiment of a toothbrush including a fingerprint sensing module.

### Detailed Description of Examples of the Invention

Exposing the mouth to light in the violet and/or blue region of the visible spectrum, between 400nm and 500nm, can be useful for a variety of purposes, including destroying bacteria and accelerating the whitening effects of a tooth bleaching agent. However, direct eye exposure should be limited in order to prevent eye damage. Thus, an aim of the present invention is to determine whether an oral care instrument is within the user's mouth or outside of the user's mouth. If the instrument is within the user's mouth, then a light emitting source such as an LED is turned on to emit light. However, if the instrument is outside the user's mouth, the LED shuts off to prevent light exposure to the eyes.

Aspects of the disclosure are illustrated in the remainder of this disclosure with reference to a manual or electric motorized toothbrush, although it is understood that the operation of any number of light-emitting oral care instruments, together with the associated advantageous features and/or beneficial effects described herein, could likewise be achieved. Other oral care instruments may include those used in dental curing lamps, oral flossing implements, and oral surgical instruments, etc.

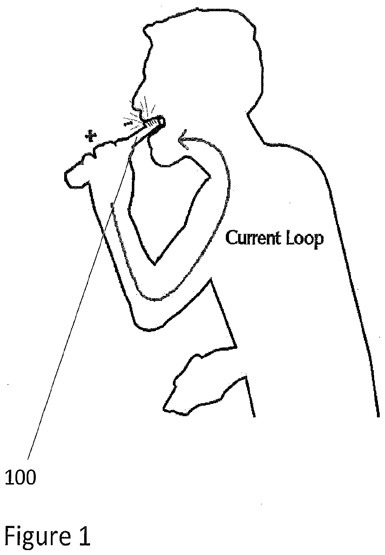

In one embodiment, as shown in Figure 1, a light-emitting oral care instrument 100 activates upon the completion of an electrical circuit between the brush handle in contact with the user's hand and contact of the brush head with the user's mouth.

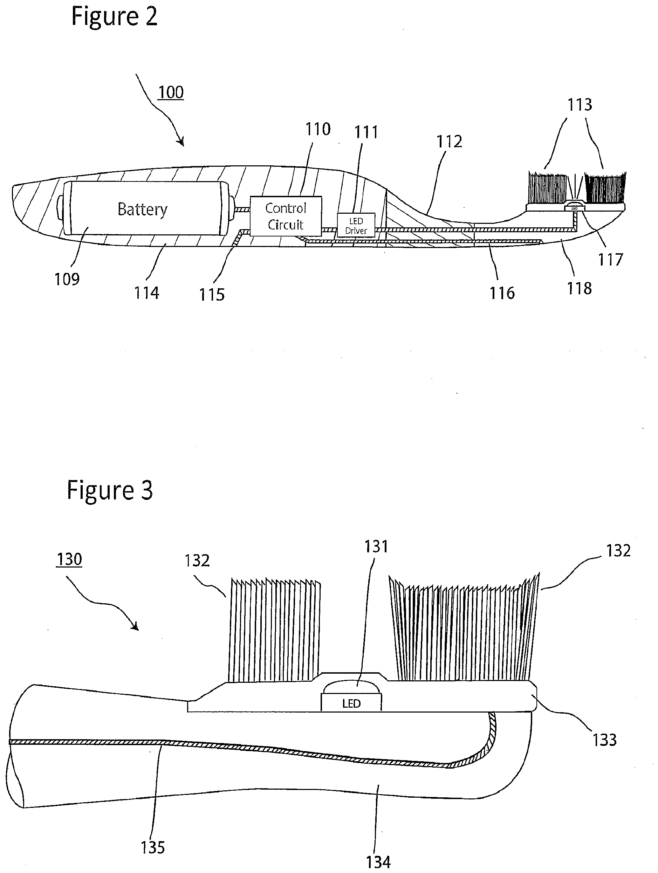

In the embodiment of the light-emitting toothbrush 100 shown in Figure 2, the brush head 118 and the handle 114 are injection molded of an electrically-conductive plastic with a non-conductive hydrophobic plastic spacer 112 located between the brush head 118 and the handle 114. The nonconductive plastic spacer 112 electrically insulates the brush head 118 and the handle 114 from each other. The brush head 118 and handle 114 are electrically connected, via a lead 116, to a control circuit 110 that can produce a low level electrical signal. While DC current is likely easiest with a battery source, the signal could be AC. The control circuit 110 can include a high sensitivity current sensor, such as Linear Technology LTC1440 Ultralow Power Single/Dual Comparator with Reference located on the control circuit 110. That comparator is available from Linear Technology Corporation, 1630 McCarthy Blvd., Milpitas, CA 95035-7417 1 (408) 432-1900, linear-tech.com.

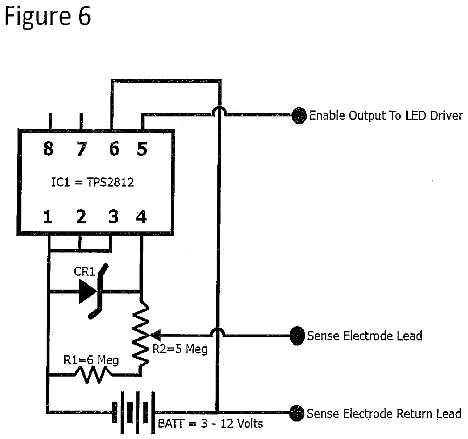

Alternatively, the signal loop sensor circuit can be the one seen in Figure 6, implemented with off the shelf components. An integrated circuit (IC1) shown in Figure 6 can be a TPS2812 Dual High-Speed Mosfet Driver available from Texas Instruments, Inc. of Dallas, Texas. One of the sensor electrodes 116 is connected through a battery supplying from three to twelve volts, to pins 1-3 and 6 of IC1. The other sensor electrode 115 is connected across and adjustment bridge variable resistor R2 and to pin 4 of IC1 across resistor R1 to pins 1-3. Pin 4 is tied through Zener diode CR1 to pins 1-3. Pin 5 of IC1 is tied as the output to the LED driver. When the signal loop through electrodes 115 and 116 is completed, the current is sensed in IC1, outputting a signal on pin 5 to activate the driver for the LED.

Prior art toothbrush sensors were implemented with older bipolar transistor technology and required high currents in the signal loop and higher voltages, producing undesirable tingling sensations for the user. The preferred signal loop sensor uses ultra-low-power CMOS technology to reduce the detection current threshold to a sub-microampere level at around one volt of potential difference, preventing any tingling sensation. Another advantage is an ultra-low battery consumption current.

A signal loop is formed through the body of the user by holding the brush handle 114 and placing the brush head 118 or wet bristles of the brush 113 in contact with the mouth. A voltage across the handle and the brush head results in a small signal current that flows through the loop and is detected by the current sensor. The current sensor outputs a signal through the control circuit 110 to the LED driver 111, which delivers current to the LED 117 within the user's mouth, causing the LED 117 to illuminate the mouth of the user. The LED preferably emits short wave length light in the band between 400-500 nm, more preferably 400-450 nm, and more preferably yet of 400-420 nm.

In an alternate embodiment 130 shown in Figure 3, conductive plastic is used in the part 133 of the brush connecting the bristles 132 to a sheet of conductive plastic 133, instead of the entire brush head. Alternatively, but also possibly in combination, as shown in Figure 3, the bristles 132 of the brush may also be conductive. When wet bristles 132 come in contact with the mouth of the user during normal brushing operations a circuit is formed via a sense electrode 135 connected to the control circuit 110, which can be used to detect the completion of the electrical signal loop and signals the driver 111 to supply electricity to the LED 131. The remainder of the brushhead 134, is composed of non-conductive plastic.

Typically when brushing teeth, a user will grasp the handle 114 of toothbrush 100, apply toothpaste to the bristles 113 and place the brush head 118 in the mouth and proceed to brush their teeth. While the toothbrush handle 114 is in contact with the user's hand and the brush head 118 is located in the mouth of the user, contact between the electrically conductive plastic of the brush head 118 and mouth of the user completes the signal loop, enabling a small DC or AC current (sensor current) to be initiated and detected by the control circuit 110. The receipt of the sensor current is signaled to the LED driver which, in turn, controls illumination of the LED 117, in this case, turns on the LED 117. When contact between the brush head 118 and the mouth is broken, i.e., when the brush head 118 is withdrawn from the mouth, the signal loop is broken, the flow of sensor current stops, and the LED 117 is turned off. It is to be appreciated that the sensor current required to control the LED 117 will preferably be in the 10 and 100 nanoampere range and would only be detectable with a high sensitivity current meter associated with the control circuitry 110. Due to the use of such a low electrical current, the user will be safe from harm and will not be exposed to danger. The galvanic current generated when a person touches a wet metal object is far greater than the current used in our application, so the current passing through the user's body is harmless and imperceptible.

The electronics, i.e. the LED driver and control circuitry 110 are configured to regulate or adjust the sensor current so that the brush head 118 must be in contact with the user's mouth to turn on the LED 117. Merely contacting the dry skin from hand to hand will not turn the LED 117 on since the dry hand is not as conductive as the wet mouth. This reduces the possibility of turning the LED 117 on merely by handling the toothbrush 100 with bare hands and inadvertently activating the LED 117 while the brush head 118 is not in the user's mouth.

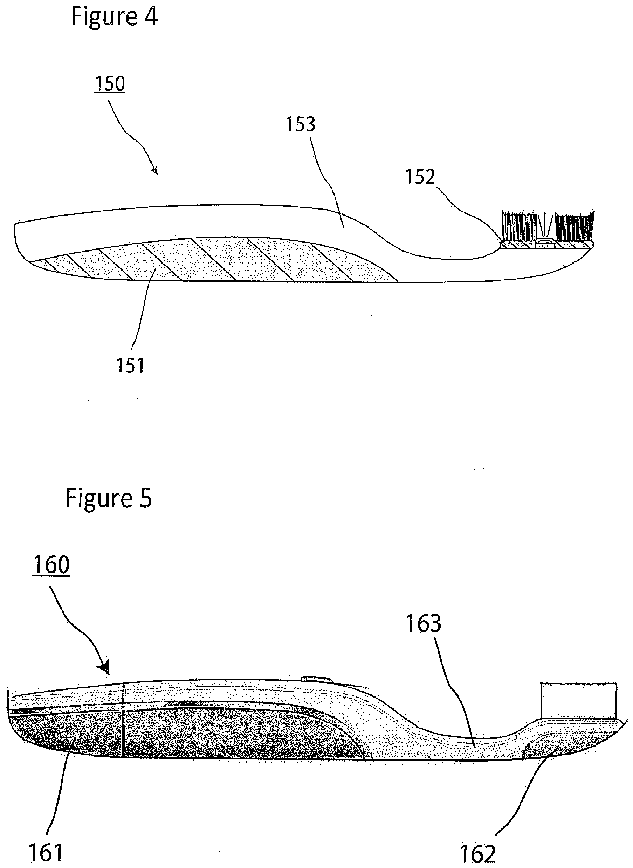

In a further embodiment of the toothbrush 150 as illustrated in figure 4 which is similar to the prior embodiment, the brush head and handle 153 could be formed from a hydrophobic, nonconductive plastic instead of the electrically conductive plastic. In this embodiment, electrically conductive metal pads 151, 152 are located on the exterior surface of the toothbrush 150. One or more metal pads 151 are located on the handle 112' and one or more metal pads 152 are located on the brush head. The metal pads 151 are positioned on the toothbrush 150 for optimal contact with the skin of the hand and pad 152 is mounted for contact with the mouth or bristles of the toothbrush. When the wet tips of the brush bristles are in contact with incisor teeth or mouth and the pad 151 is in contact with the user's hand, an electrical circuit is formed through the body of the user. It is to be understood that the metal pads 152 of the brush head should be spaced apart from the metal pads 151 of the handle.

In use, while the metal pads 151 of the toothbrush handle are in contact with the user's hand and when the brush head 152 is located in the mouth of the user or wet bristles are in contact with any wet portion of the mouth or teeth, contact between the metal pads 152 of the brush head and mouth of the user completes a signal loop, enabling a small DC current (sensor current) to be initiated and detected by the control circuit 110. The sensed current causes the control circuit to activate the LED driver to turn on the LED 126. When contact between the brush head and the mouth is broken, i.e., when the brush head is withdrawn from the mouth, the signal loop is broken, the flow of sensor current stops, and the control circuit turns off.

A further embodiment 160 is shown in Figure 5. The handle has a portion 161 of conductive plastic, and the head has a portion 162 of conductive plastic. Portion 163 is non-conductive. The conductive plastic areas are connected internally to circuits as described in connection with Figure 2 and so the toothbrush of Figure 5 can be used in like manner to the toothbrush of Figure 2.

Many electric toothbrushes employ a timer to alert the user of the end of a preset brushing time, for example, two minutes, as recommended by the American Dental Association. These timer circuits are commonly combined with vibration or noise to alert the end user to the completion of a recommended brushing period. The signal loop sensor disclosed herein can be combined with such a timing circuit in a manner that causes elapsed time to be recorded only when the sensor is activated (i.e. light is on). This would facilitate the assurance that the brush timer was actually measuring elapsed time in the mouth of the user and not simply elapsed time of the manual activation of the brush. The control circuit can also include a timer to turn off the LED at a preset time, such as two minutes, signaling to the user that tooth brushing has lasted two minutes. Other ways to signal the user of the completion of two minutes can be substituted.

A conventional make/break switch can be included in one of the conductors 115 or 116 or elsewhere in the electrical circuit, if desired. Thus, the signal loop is completed only if the user closes that switch AND inserts the toothbrush head into the mouth. This can provide a further safety and convenience feature. Alternatively, a make/break switch could be located elsewhere in the circuit from the battery, to the control circuit through the LED driver to LED.

Since the LED will be on only when the light from the LED is safely and effectively used, the life of a charge on the battery should be longer.

The battery can be rechargeable or replaceable, as will be apparent to those of ordinary skill in the art.

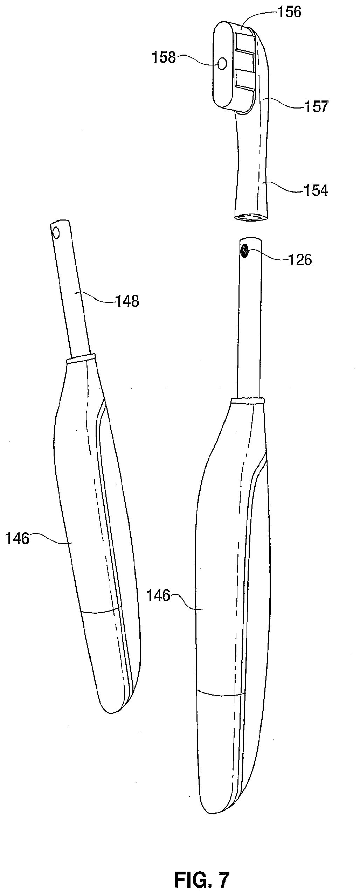

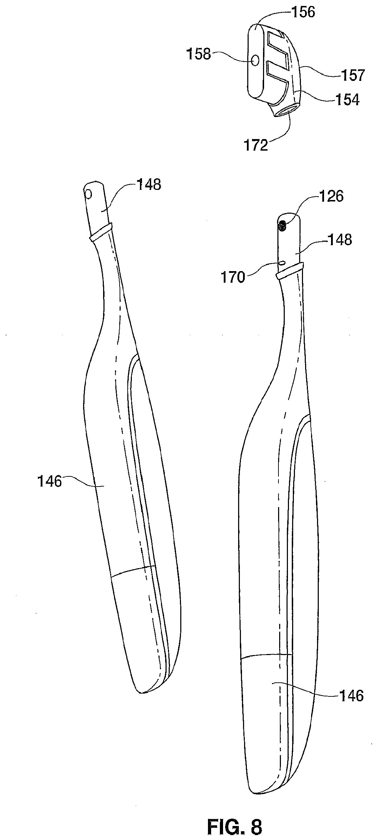

The brush head and/or neck can be replaceable, as long as the replacement part has the correct electrical contact and a conductor that can reliably connect to a mating conductor in the brush handle. The design can allow the sensor current to flow across the junction of the permanent part and the replaceable part through either two sheets of conducting plastic or a metal connector such as a pin connector. Examples are seen in Figures 7 and 8.

As seen in Figures 7 and 8, a brush handle 146 is provided having the necessary battery, charging electronics and LED, all self-contained and water-tightly encapsulated within the handle. The handle includes a stem 148 in which the LED 126 is mounted. Figures 7 and 8 show two different embodiments, with the stem 148 in Figure 7 being longer than the stem 148 of Figure 8. The replaceable brush head 157 has a hollow shaft 154 of an internal diameter slightly larger than the diameter of the stem 148, so the stem 148 can be inserted into the hollow shaft 154. The brush head has an array of bristles 156 and an opening 158 to allow the light from the LED 126 to pass toward the teeth as they are brushed by the bristles. Preferably the stem and hollow shaft have complementary, non-circular shapes so that the bristles do not rotate around the stem, but stay in a fixed orientation. When the bristles are spent, the brush head 157 can be removed from the stem 148 and replaced with a new brush head. Other appliances appropriate for a tooth whitening brush pattern, light curing of gum infections, tongue scraper, flosser, etc can be configured with similarly shaped hollow shafts so they can also be mounted onto the stem 148.

In the embodiment shown in Figure 7 the plastic of the stem 148 and the head 157 are made electrically conductive, so that the signal loop can be completed as the user puts the head 157 (as installed on the stem 148) into his or her mouth and grasps the handle 146.

In the embodiment shown in Figure 8 a conductor in the plastic of the stem 148 has an electrical contact 170; the head 157 has an electrical contact 172 within the shaft 154 positioned to mate with contact 170 when the head is mounted on the stem. The signal loop can be completed as the user puts the head 157 (as installed on the stem 148) into his or her mouth and grasps the handle 146.

A further enhancement of the interlock control feature of this toothbrush can be realized using an AC control signal in place of the DC loop sensor current. A low-frequency 200 Hz to 10 kHz square or sinusoidal waveform AC signal with a peak to peak voltage of no greater than 1 V will be applied to the toothbrush handle. This AC signal will be conducted through the user's body in much the same way as a DC current, as described above. One way to implement this is through selective tone filtering in which a Controlled Oscillator in the handle puts off a square wave form in the low audio band of 1 to 2 kHz. A narrow band tone filter only allows one tone to come through in the brush head. This Selective Tone Filter in the brush head is looking for one certain tone. When it receives that tone, it turns on the LED.

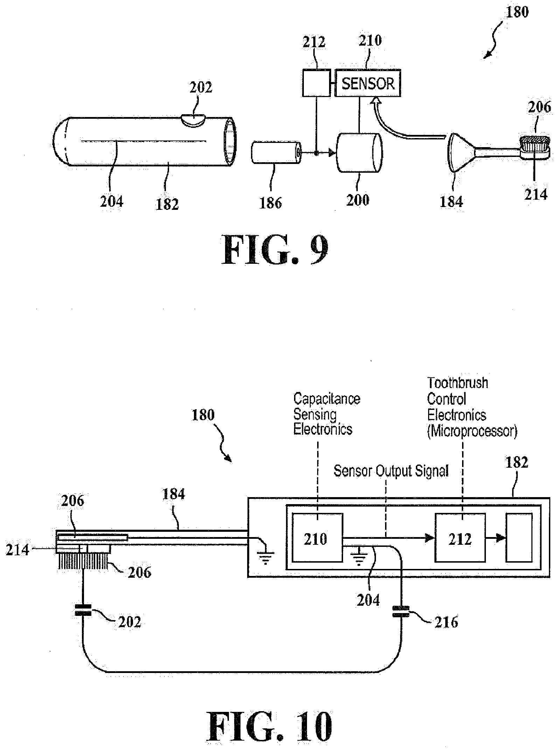

In another embodiment as shown in Figures 9 and 10, the sensor used to detect whether the oral care instrument 180 is within the user's mouth is a capacitive sensor. The oral care instrument may be a toothbrush 180 with a battery 186, a LED driver 200, and an on/off switch 202. An electronics ground 204 is located on the handle 182 of the brush. A conductive electrode sensing element 206 is installed preferably on the brush head 184. A sensor system 210 measures the capacitance 216 between the sensing element 206 and the electronics ground 204. A microprocessor/controller 212 determines whether the measured capacitance 216 is above a threshold value, which indicates that the toothbrush 180 is inside the user's mouth. If the measured capacitance exceeds the threshold value, then the LED driver 200 receives a signal and turns on the LED 214. If the measured capacitance drops below the threshold value, then the LED driver turns off the LED 214.

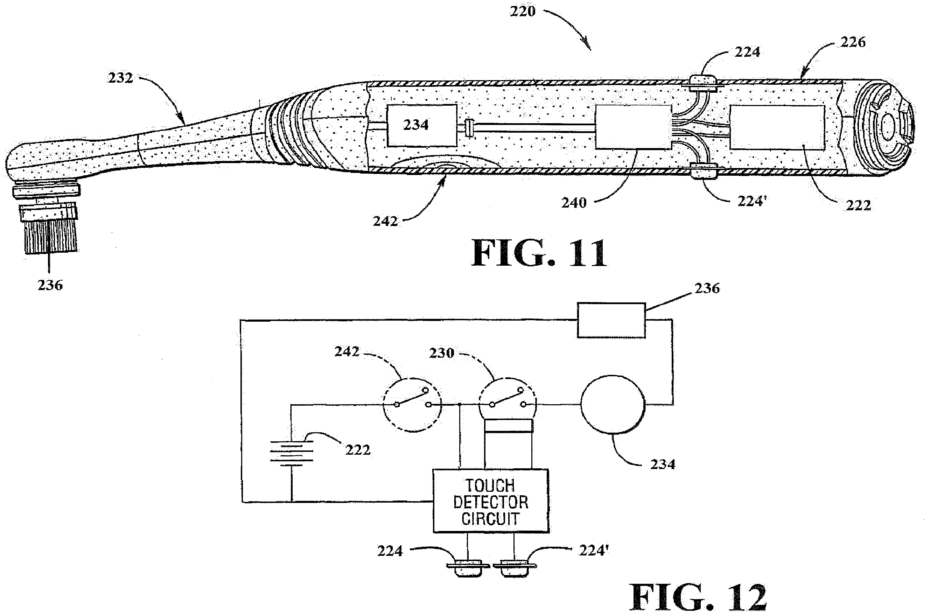

In yet another embodiment, the sensor may be a capacitive displacement sensor. One or more tactile sensors are preferably placed on the handle portion of the oral care instrument, and measure capacitance of a conductive target such as the human body. As shown in Figure 11, the oral care instrument is a toothbrush 220 with a battery 222 and tactile sensors 224, 224' placed on opposing sides of the handle 226. A switch 230 is off when in its first position. When a user grips the handle 226 at both tactile sensors 224 and 224', preferably when the brush head 232 is inserted inside the user's mouth, the capacitive sensor 240 senses an increase in capacitance. As shown in Figure 12, the capacitive sensor 240 causes switch 230 to move to its second position, signaling the LED drive 234 to turn on the LED 236. An additional on/off switch 242 operated by the user may be installed onto the oral care instrument.

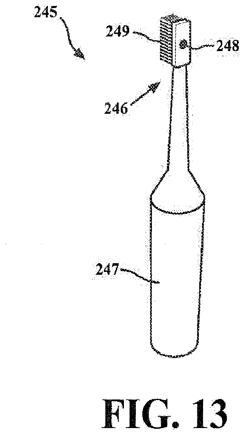

In another embodiment the sensor is an inductance proximity sensor which uses a radio frequency loop to sense the proximity of conductors such as the human body. In one embodiment, as shown in Fig. 13, the dental hygiene implement is a toothbrush 245 having a brush head 246 and handle 247. In this embodiment, the proximity sensor 248 is on the back of brush head 246. The inductance proximity sensor 248 is comprised of an LC oscillating circuit with a signal evaluator. The LC oscillating circuit is comprised of a coil and a capacitor that emits a highfrequency electromagnetic alternating field at the sensing face of the sensor. When the brush head is inserted into the user's mouth, Eddy currents are generated that reduces the oscillations within the LC oscillating circuit. The signal evaluator detects this change in oscillation frequency, and the resulting reduction in oscillations sends a signal to the LED driver to turn on LED 249. When the brush head is removed from the user's mouth, the oscillations from the LC oscillating circuit return to normal and the LED driver turns LED 249 off. Different types of inductor circuits may be used in other embodiments of the present invention.

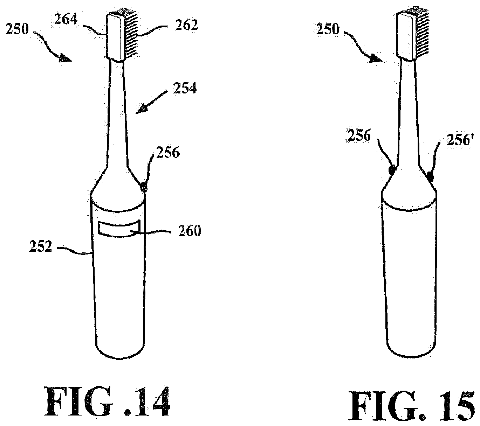

In another embodiment the sensor is a passive thermal infrared sensor. The passive thermal infrared sensor may be placed anywhere on an oral care instrument. In the embodiment shown in Figures 14 and 15, the oral care instrument is a toothbrush 250 with a handle 252 and brush head 254. At least one passive thermal infrared sensor 256 is placed near the upper portion of the handle 252 toward the brush head 254. As the user inserts the brush head into the mouth, the microprocessor 260 detects a change in infrared light as detected by the passive IR sensor 256. The resulting increase in IR light causes the microprocessor 260 to turn on LED 262. As the user removes the brush head out of the mouth, the resulting decrease in IR light sensed by the passive IR sensor 256 causes the microprocessor 260 to turn off LED 262.

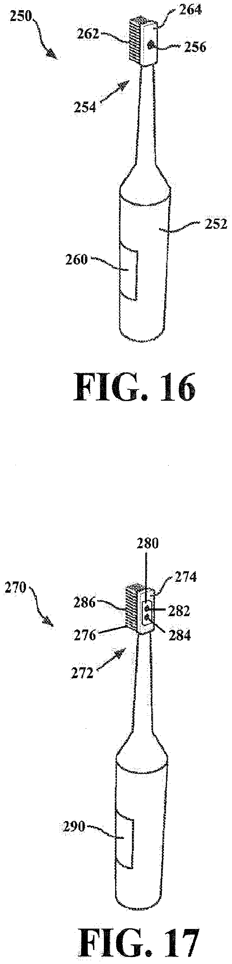

In another embodiment, the passive thermal infrared sensor is placed on the brush head as opposed to the handle. In the embodiment shown in Figure 16, the passive IR sensor 256 is placed on the back of the bristle plate 264. Like the embodiment in Figures 14 and 15, if the user places the brush head in his mouth, then the microprocessor 260 will turn on LED 262 due to the increase in IR light as sensed by passive IR sensor 256. As the user removes the brush head out of the mouth, the resulting decrease in IR light sensed by the passive IR sensor 256 causes the microprocessor 260 to turn off LED 262. The change in infrared light as the brush head enters the mouth is understood to be a result of the change in temperature. Thus, it is understood that the passive IR sensor disclosed herein may be substituted with other means for detecting changes in temperature.

In another embodiment the sensor is an active thermal infrared. This sensor uses a photoelectric sensor that detects reflected IR light emitted and absorbed by the sensor itself. This could be used to detect proximity inside the mouth. The active thermal IR sensor 280 is typically comprised of a light source 282 that emits IR light and a photo diode 284 that detects changes in the sensing environment as the IR emitted from the light source is redirected. In one embodiment, an active thermal infrared sensor is placed on the brush head 272 of a toothbrush 270. The active thermal infrared sensor may be placed either on the front face of the plate with the bristles 276 and LED 286. Alternatively, as shown in the embodiment in Figure 17, the active thermal infrared sensor 280 may be placed on the back of the bristle plate 274. If the user places the brush head in his mouth, then the microprocessor 290 will turn on LED 286 due to the change in IR light as sensed by photo diode 284. As the user removes the brush head out of the mouth, the IR light returns to a default value as detected by photo diode 284 which causes the microprocessor 290 to turn off LED 286.

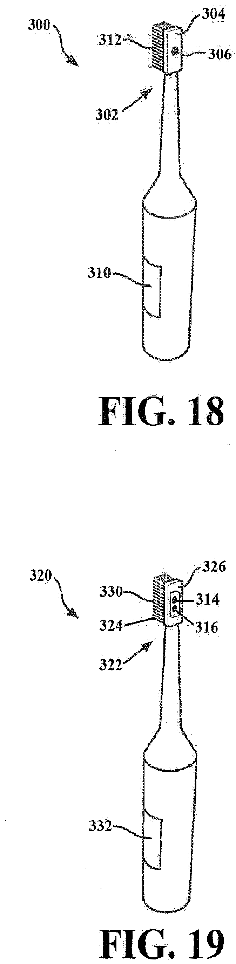

In another embodiment the sensor is a passive optical sensor where the darkness caused by inserting the oral care instrument into the mouth triggers the short wavelength LED to turn on. Figure 18 shows an embodiment wherein the passive optical sensor is installed on a toothbrush 300. In this embodiment, the passive optical sensor 306 is preferably on the brush head 302 of the toothbrush. In the embodiment shown in Figure 18, the passive optical sensor 306 is placed on the back of the bristle plate 304. If the user places the brush head in his mouth, then the microprocessor 310 will turn on LED 312 due to the decrease in light as sensed by passive optical sensor 306. As the user removes the brush head out of the mouth, the resulting increase in light sensed by the passive optical sensor 306 causes the microprocessor 310 to turn off LED 312.

In another embodiment the sensor is a photocell 314 that detects the reflection of light from a second light source 316 on the oral care instrument. In one embodiment, a photocell is placed on the brush head 322 of a toothbrush 320. The photocell 314 and accompanying light source 316 may be placed either on the front face of the plate with the bristles 324 and LED 330. Alternatively, as shown in the embodiment in Figure 19, the photocell 314 and secondary light source 316 may be placed on the back of the bristle plate 326. If the user places the brush head in his mouth, then the light emitted by light source 316 will reflect and be detected by the photocell 314. As the reflected light is detected by photocell 314, the microprocessor 332 will turn on LED 330. As the user removes the brush head out of the mouth, the photocell 314 will no longer detect the reflected light which causes the microprocessor 332 to turn off LED 330.

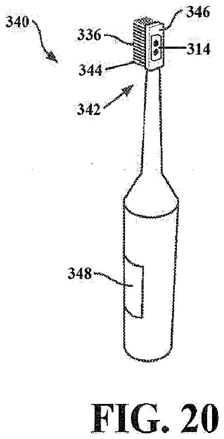

In another embodiment the sensor is an ultrasonic sensor that emits sound waves and uses echo location to detect whether the oral care instrument is within the confines of the mouth. In one embodiment, an ultrasonic sensor 334 is placed on the brush head 342 of a toothbrush 340. The ultrasonic sensor 334 may be placed either on the front face of the plate with the bristles 344 and LED 336. Alternatively, as shown in the embodiment in Figure 20, the ultrasonic sensor 334 may be placed on the back of the bristle plate 346. If the user places the brush head 342 in his mouth, then the duration that it takes for the sound emitted by ultrasonic sensor 334 to be received back will decrease. The decrease in durational response will cause the microprocessor 348 will turn on LED 336. As the user removes the brush head out of the mouth, the duration that it takes for the sound emitted by ultrasonic sensor 334 to be received back will increase causing the microprocessor 348 to turn off LED 336.

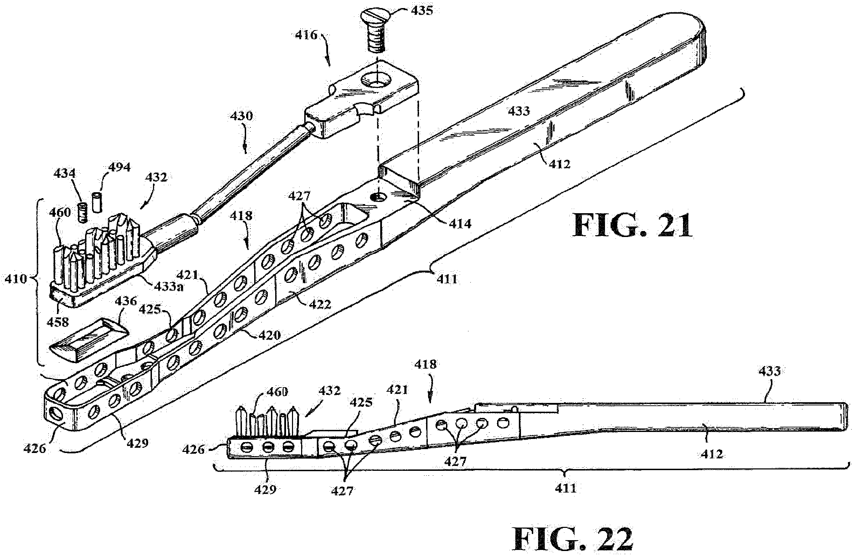

In another embodiment the sensor is a pressure sensor under the brush head that detects movement and pressure of the brush head being pressed against the teeth. Figure 21 shows the pressure sensing system 410 of the present invention 410 and a toothbrush body referred to generally at 411. Generally, toothbrush body 411 will be a manual toothbrush. The toothbrush body 411 includes a handle portion 412, which is configured to be grasped by the hand of the user. In the embodiment shown, handle 412 is approximately 9.5 cm (3.75 inches) long, while the entire toothbrush body 411 is approximately 18.8 cm (7.5 inches) long. In the embodiment shown, toothbrush body 411 is made of a filled nylon, but could be other materials as well, including polypropylene and other plastics. Handle portion 412 is in the embodiment shown approximately 1.2 cm (0.5 inches) wide and approximately 0.94 cm (⅜ inches) high. The handle portion 412 is closed about all four sides and its two ends.

At the distal end of handle 412 is a portion 414 which in the embodiment shown is adapted to receive a hinged member portion 416 of the pressure sensing assembly 410. The remaining portion of the toothbrush body is referred to at 418, and is generally U-shaped in cross-section, open at the top. The remaining portion 418 comprises a base 420, two upstanding sides 421, 423 and a forward end wall 426. This arrangement provides rigidity for the toothbrush body. From the receiving portion 414, toothbrush body 411 begins to taper inwardly at both sides over a short distance until the width of the toothbrush body is approximately 0.6 cm (0.25 inches). Over this distance, the top edges of the sides 421, 423 toothbrush body are flat for a small distance and then angle downwardly until point 425 on the toothbrush body. Over this distance, base 420 angles slightly downwardly. The drawings show this structural arrangement, in particular Figure 22.

From point 425 to forward end wall 426, the toothbrush body is flat and is adapted to receive a conventional toothbrush brushhead 432. The distance from the lower surface 429 of the flat section 431 to the upper surface 433 of handle portion 412 is approximately 1.87 cm (0.75 inches), while the height of the toothbrush body in the flat section 431 is approximately 0.70 cm (0.28 inches).

The sides 421 and 423 and the base 420 over the length of the toothbrush body from receiving portion 414 to the forward end wall 426 have a plurality of openings 427-427 therethrough. In the embodiment shown, these openings are circular, approximately 3 mm (0.125 inches) in diameter, spaced approximately 0.6 - 0.88 cm (0.25 - 0.35 inches) apart. In base 420 of flat section 431 is an elongated slot 437, which is discussed in more detail below. The openings could have other shapes and spacing, however. The use of openings, with an entirely open top, has several advantages. It allows fluid to easily escape the brush, without trapping oral tissue in the openings. This arrangement further permits the use of the hinged arm pressure sensing assembly 411 without the use of seals between the arm and the body. The openings further are large enough to not only allow rinsing water to move freely in and through the toothbrush body during cleaning, but also allows the unit to dry out thoroughly between uses.

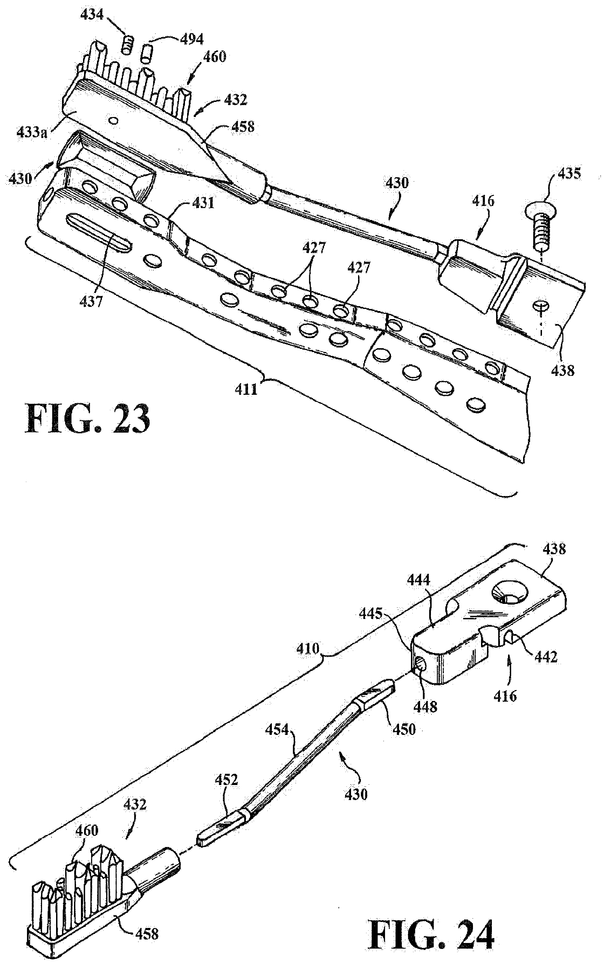

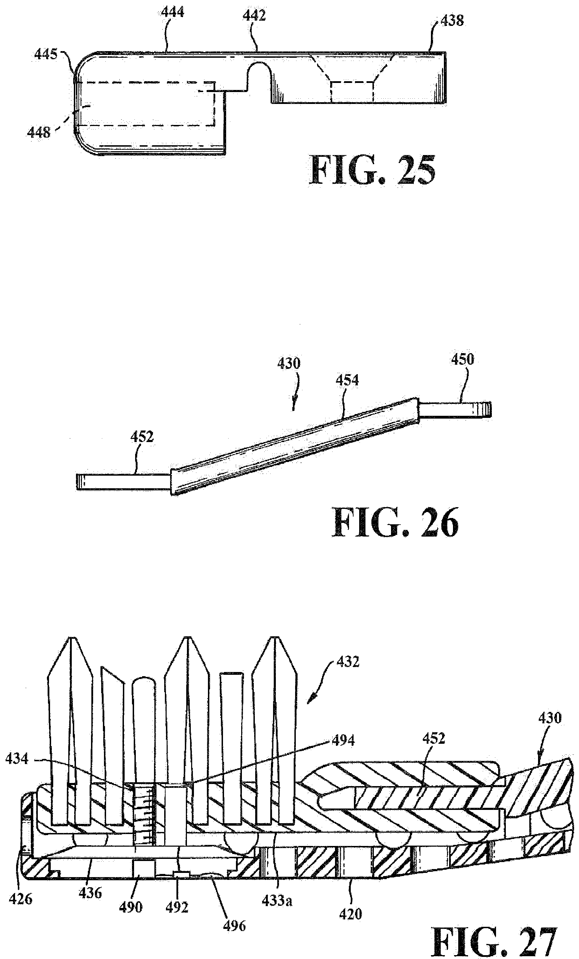

The pressure sensor assembly 410 is shown in relation to the toothbrush body 411 in Figure 21, and in an exploded view by itself in Figure 24. Two of the component parts thereof are furthermore shown in more detail in Figures 25 and 26. The pressure sensor assembly/system includes a hinged member 416, an elongated arm 430, a brushhead 432 which includes a striking element 434 extending away from a rear surface 433 of the brushhead, and a deformable dome element 436, conventionally referred to as a "snappy" member, since it makes a snap-like sound when deformed past a threshold point. Most round snap domes cannot be moved beyond a "flat" position without turning inside out. The rectangular snap dome shown and described herein can be moved to a "beyond flat" position, thereby providing a longer collapsing distance and greater tactile feel.

Hinged member 416 is attached to toothbrush body 411 at receiving portion 414, by means of a screw 435 or the like. It could also be a quick disconnect arrangement to allow convenient replacement of the brushhead. Hinged member 416 in the embodiment shown is made from polypropylene or acetal resin (Delrin) or similar plastic. The hinged member 416 (Figure 25) includes a rear portion 438 which is approximately square in the embodiment shown and approximately 3 mm (⅛ inch) thick. Forward of base portion 438 is a narrow hinge portion 442 which in the embodiment shown is approximately 0.38 mm (0.015 inches) thick, which is sufficiently thin to permit a hinge-like action, and approximately 1.25 cm (0.5 inch) wide.

Forward of hinge portion 442 is a receiving portion 444, which is approximately 0.6 cm (0.25 inches) thick. The receiving portion 444 is approximately 0.8 - 1.25 cm (0.3 - 0.5 inches) wide at hinge portion 442 and tapers to approximately 0.8 cm (0.3 inches) at a forward end 45 thereof. The longitudinal edges of the receiving portion 444 are in the embodiment shown rounded. The receiving portion 444 is configured to fit within the toothbrush body, near a rear end of the remaining open portion 418 thereof. An octagonal (in cross-section) central opening 448 extends longitudinally inward of receiving portion 444 from forward end 445 and receives one end of an arm 430.

Elongated arm 330 in the embodiment shown (Figure 24) includes proximal and distal portions 450 and 452, connected by an intermediate rod-like portion 454. In the embodiment shown, arm 430 is made from stainless steel, but other materials could be used as well, such as various plastic materials. Proximal portion 450 is approximately 1 cm (0.4 inches) long and is configured to snugly fit into opening 448 in receiving portion 444 of the hinged member, while distal portion 452 upon which brushhead 432 is mounted is approximately 1.25 cm (0.5 inches) long. The intermediate portion 454 is approximately 3.32 cm (1.328 inches) long in the embodiment shown.

The arm 430 has a total length of 5.612 cm (2.245 inches), because the intermediate portion is arranged such that it angles downwardly between the proximal and distal portions. The distance between the centerlines of the proximal and distal portions is approximately 0.87 cm (0.35 inches). The angle of the intermediate portion of the embodiment shown is approximately within the range of 5°-20°, preferably 15°. The intermediate portion 454 is configured to closely follow the portions of the toothbrush body in which it fits.

Although the hinged member 416 and arm 430 are shown as two pieces in the present embodiment, they could be made, i.e. molded, as a single unit.

Mounted on distal end portion 452 of arm 430 is a brushhead 432. Brushhead 432 includes a base portion 458 and a bristle portion 460 which is mounted in base 458 and extends upwardly therefrom in conventional fashion. The bristle portion can take various configurations, including conventional arrangements or special configurations to accomplish particular brushing effects. In the arrangement shown, the tops of the bristles are in approximately the same plane as the hinge portion 442 of the hinged member to prevent in/out brushing forces at the bristle tips from causing turning moments around the hinge member and distorting the accuracy of the force sensing system. The combination of the hinged member 416, arm 430 and brushhead 432 can be replaceable as a unit if desired.

Mounted in the base of the brushhead, approximately central thereof in the embodiment shown, is a set-screw which is the striking element 434. The set-screw extends through the base portion 458 and below the lower surface 433 of the brushhead, approximately 2 mm (0.08 inches) in the embodiment shown. The setscrew in the embodiment shown is approximately 2.3 mm (³/₃₂ inches) in diameter and 3 mm (⅛ inch) long and is made from stainless steel. Alternatively a bump could be molded into the toothbrush base portion 458. Further, the hinged element, the arm and the brushhead could be a single piece. The brushhead 432 could also be made removable from the arm portion.

When hinged member 416 is secured to the receiving portion 414 of the toothbrush body, application of force against the brushhead 432 toward the toothbrush body will result in the brushhead moving about hinged portion 442 of hinged member 416.

A thin dome element 436 is secured to interior surface of flat section 431 of the toothbrush body, directly beneath base portion 458 of the brushhead. Dome element 436 in the embodiment shown is a conventional snap dome member having an obround configuration, similar generally to a child's "cricket" toy. The obround snap dome element 436 is capable of moving "beyond flat" when it suddenly collapses due to pressure against it exceeding a particular value by action of the striking element 434. This is shown by the dotted lines in Figure 27. The "beyond flat" capability, as discussed above, is important to provide a sufficient collapsing distance that the user can recognize the collapse of the element. The snap dome is mounted on a ridge within the brushhead receiving portion to permit the center portion of the snap dome element to go beyond flat.

In the embodiment shown, the dome element collapses approximately 16 mm (1/16 inch) and beyond flat by approximately 11 mm (0.045 inches). The force necessary to collapse the dome is preferably under 200 grams, since selected force values above this range are generally considered as a threshold for excessive pressure. Snap dome elements are available with various collapse forces.

By default, a switch 490 is in an "off state. In one embodiment, when the snap dome 436 collapses it mechanically triggers the switch 490 to an "on" state. Turning the switch onto its "on" state closes the circuit containing battery 496 and causes the LED driver 492 to turn on the LED 494. While the snap dome 436 remains collapsed due to the pressure applied when a user brushes his teeth, the LED 494 remains on. Other types of switches may be used, including a magnetic sensor where the striking element 434 causes a first magnetic plate to come into contact with a second magnetic plate.

The snap dome is secured to the toothbrush body beneath the brushhead by means of an adhesive or tape or a trapping element. Slot 437 (Figure 23) in the base portion of the toothbrush body extends beneath the snap dome, and prevents the possible damping of the snap dome action due to fluid being trapped beneath the dome when it collapses. The slot allows the ready escape of the fluid from the toothbrush body and allows for complete rinsing and drying of the toothbrush between uses.

In the embodiment shown, the snap dome element is secured to the toothbrush body beneath the brushhead and the striking element extends from the brushhead. In another embodiment, the snap dome element could be positioned on a lower surface of the brushhead and the striking element could be positioned on the toothbrush body beneath the brushhead.

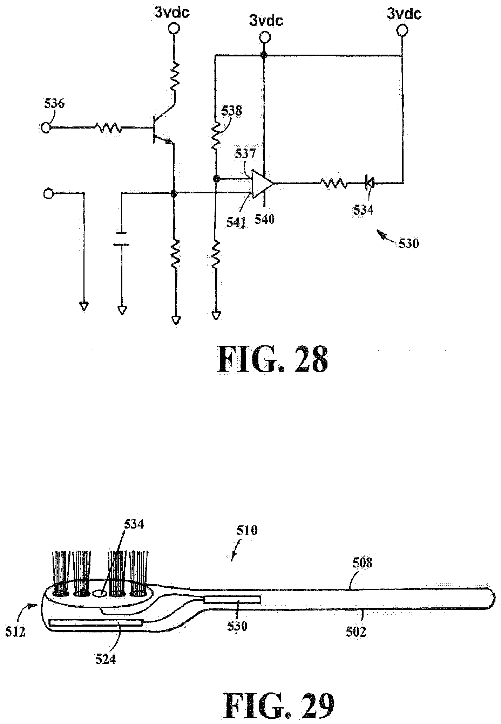

Other pressure sensors may be used. Toothbrush 510 for sensing pressure applied during brushing and for indicating when the pressure exceeds a predetermined value is shown in partial cross-section in FIG. 29. Toothbrush 510 includes a piezoelectric film 524 disposed within brush head 512 and an indicator circuit 530, including LED 534, disposed within handle 508.

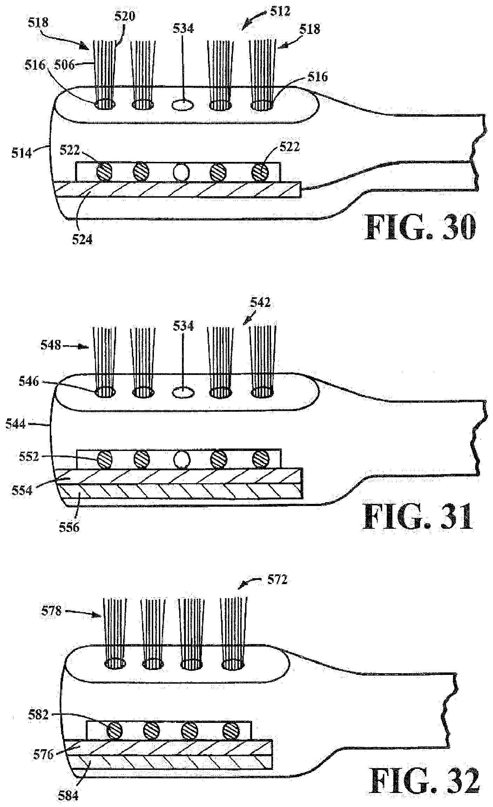

Referring to FIG. 30, an enlarged cross-sectional view of brush head 512 is shown. Tufts 518 of bristles 506 extend through openings 516 in member 514 of head 512. Each tuft has a crest end 520 extending outward from opening 516 and a root end 522 disposed beneath opening 516 in member 514. The length of bristles extending between crest end 520 and root end 522 of tuft 518 has a cross-sectional dimension that is less than the cross-sectional dimension of opening 516 so that tuft 518 can move axially through opening 516 and retract downwardly toward piezoelectric film 524.

The cross-sectional dimension of root end 522 is greater than the cross-sectional dimension of opening 516, which inhibits root end 522 from passing through opening 516 and retains tuft 518 within head 512.

Piezoelectric film 524 is disposed within head 512 beneath root ends 522 of tufts 518 so that film 524 will experience strain when pressure is applied to tufts 518. When pressure is applied to tufts 518, root ends 522 are forced into contact with piezoelectric film 524. The pressure exerted by root ends 522 on film 524 causes a change in the strain of film 524, which causes film 524 to generate voltage. As is well known in the piezoelectric art, piezoelectric films carry a permanent dipole moment that, when the film is at rest, is cancelled out by charges in the atmosphere. Deforming the film, i.e., applying a force to the film that generates a strain, changes the orientation of the polymer backbone of the film, which causes the strength of the dipole to change and generates an electrical voltage. If the piezoelectric film comes to rest in its deformed position, i.e., the pressure applied is constant and no new strain is generated, the new dipole will again be cancelled out by atmospheric charges and the voltage will cease. As long as the orientation of the polymer is being changed by application of a varying degree of pressure to the film, voltage will be generated.

Referring to FIGS. 28 and 29, indicator circuit 530, located within the handle 508 of toothbrush 510, senses the voltage generated in piezoelectric film 524 and determines whether the voltage generated is greater than a predetermined value, e.g., a value that represents a level of brushing pressure. If the voltage is greater than the predetermined value, then indicator circuit 540 turns LED 534 on. If the voltage drops below the predetermined value, then the indicator circuit 540 turns LED 534 off. Any circuit capable of receiving a signal from the piezoelectric film, determining if the signal is greater than a predetermined value, and subsequently turning on the LED 534 can be used as the indicator circuit. Such circuits can be readily constructed by those skilled in the art.

One example of a suitable indicator circuit is shown in FIG. 28. The voltage generated in piezoelectric film is transferred via lead 536 to an indicator circuit 530 that includes comparator 540 and a single LED 534. LED 534 turns on when the output voltage from the piezoelectric film (applied to variable input 541 of comparator 540) exceeds the voltage drop across resistor 538 as applied to reference input 537 of comparator 540.

The LED of the indicator circuit is preferably located on the toothbrush in such a way that it is not seen by the user when brushing. Thus, the LED is preferably located at the brush head 512.

Referring to FIG. 31, an alternate embodiment of toothbrush head 542 is shown in which a resiliently deformable membrane material 556 capable of resilient spot deformation, i.e, a membrane that has highly localized zones which can be resiliently displaced relative to the rest of the membrane without affecting the zones immediately adjacent to the displaced zone, is disposed beneath piezoelectric film 554. Such a membrane material is described, e.g., in U.S. Pat. No. 4,633,542 (Taravel).

When the toothbrush is at rest, i.e., when no pressure is applied to tuft 548 urging it against piezoelectric film 554, membrane 556 is taut and resiliently biases root end 552 of tuft 548 against member 544. When pressure is applied to tuft 548, tuft 548 retracts into contact with piezoelectric film 554 by sliding through opening 546 and causing resilient spot deformation of membrane 556 at the point where tuft 548 is urged against piezoelectric film 554, without displacing the contact zones between the root ends of the respective immediately adjacent tufts and the membrane. Once the strain is released from the membrane, the membrane material regains its original structure. When the toothbrush is removed from the oral surface, i.e., the pressure is removed, tufts 548 return to their initial position with their respective root ends 552 abutting against member 544 by virtue of resilient membrane 556 returning to its initial position. Membrane 556 is preferably under tension since tension facilitates this spot deformation.

Membrane 556 is preferably formed from an isotropic elastomeric material selected for its ability to behave anisotropically in that it gives the membrane the property of being able to exhibit resilient deformation in localized spots. The choice of material for the membrane and the appropriate thickness are readily determined by the person skilled in the art as a function of the mechanical characteristics and, in particular, the elasticity required for the membrane. Suitable membrane materials include, e.g, natural or synthetic latex type elastomers (e.g., polychloroprenes), natural rubber, and silicones.

The thickness of the membrane in the relaxed state will generally vary in the range of 0.10 mm to less than 1 mm. Such a thickness will enable localized or spot resilient deformation with an amplitude of 0.5 mm to 5 mm for a force of about 1 Newton (N) to 7.5 N (i.e., about 150 grams-force to about 750 gf) applied in a distributed manner over the set of tufts of bristles.

Alternatively, membrane 576 may be disposed above piezoelectric film 584 between root ends 582 of tufts 578 and piezoelectric film 584, as shown in FIG. 32.

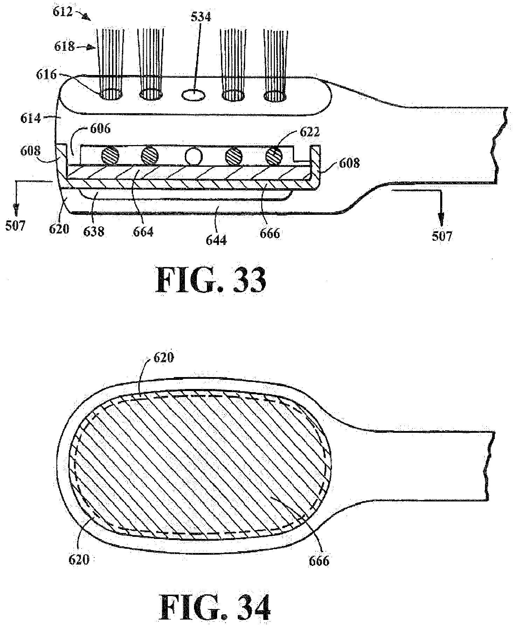

Referring to FIG. 33, in another embodiment, membrane 666 extends across a cavity 638 defined by member 644 in head 612 so as to seal cavity 638. The toothbrush also includes piezoelectric film 664. During assembly of the toothbrush membrane 666 is fixed, while taut, to head 612 at the periphery of cavity 638. Cavity 638 and membrane 666 enclose a cushion of air, the presence of which facilitates vertical spot deformation of membrane 666 in response to axial retraction of a tuft of bristles. Member 614, which is made of rigid material, e.g., a plastic material, like the remainder of the brush head, is fixed to the brush head and together therewith clamps the membrane in continuous manner all around the periphery of cavity 638. Member 614 may be a separate piece forming a rigid extension of the head or may be integrally molded with the head.

A continuous rim 606 projects substantially perpendicularly from an area near the perimeter of member 614. A continuous peripheral zone 608 of membrane 666 is clamped between the side of head 612 and rim 606 thereby fixing membrane 666 to the head and ensuring that membrane 666, member 614 and head 612 are fixed relative to one another. In addition or alternatively, membrane 666 may be affixed to rim 620 around the edge of cavity 638, e.g., by glue or a heat weld, as shown in FIG. 34.

Any method may be used to fix the periphery of the membrane to member 614 or rim 620, provided the portion of the membrane that contacts the root ends 622 of tufts 618 remains resiliently deformable. Tufts 622 retract into and are biased by membrane 666 in the same manner as discussed above with reference to FIGS. 31 and 32.

Other exemplary embodiments include, for example, an embodiment in which the piezoelectric film extends across a cavity, e.g., the cavity shown in FIG. 33, in the absence of a membrane. In another embodiment, the indicator circuit is located external to the toothbrush.

In another embodiment the sensor is a pressure sensor in the neck that detects torque and tension in the neck of the brush due to brushing action. The pressure sensor could be Piezoelectric, Capacitive, Potentiometric, Optical or Electromagnetic. Such a pressure sensor is disclosed in US 20030205492 to Ferber et al.

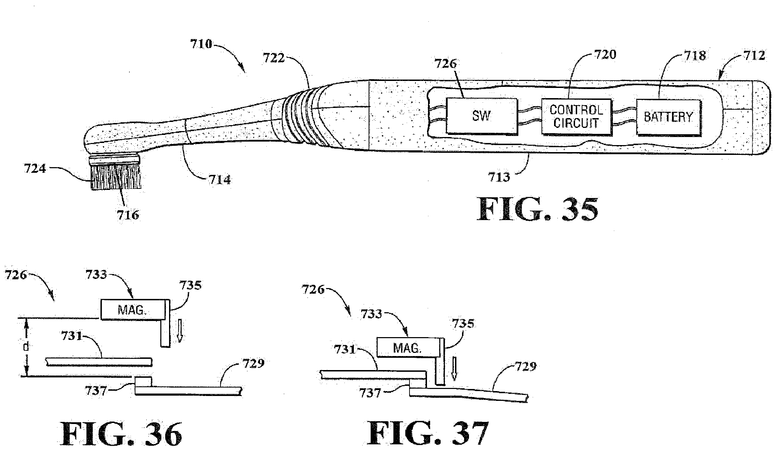

FIG. 35 shows a simplified descriptive view of a light emitting toothbrush 710. The toothbrush 710 includes a toothbrush body 712 that has a handle portion 713 and a brush head portion 714. Within the brush head portion 714 is light source 716. The light sources 716 are powered by a battery 718, and controlled by electrical circuitry, or a control circuit 720. Although only one battery 718 is illustrated in FIG. 35, it is contemplated that more than one battery may also be used. The toothbrush body 712 includes a flexible portion 722 that facilitates some movement of the brush head portion 714 when a force is applied to the brush head portion 714, for example, when bristles 724 are applied to an operator's teeth. A switch 726, which is configured to activate the light source 716, is actuated when a first predetermined force is applied to the brush head portion 714.

The interaction of the switch 726 and the movement of the brush head portion 714 exemplifies one of the benefits of the toothbrush. It has been shown that effective brushing occurs when a force of 2N-3N is applied in a direction normal to the teeth. When the brushing force is significantly less than 2N, cleaning of the teeth may not be adequate. When a brushing force of significantly more than 3N is applied to the teeth, unacceptably high levels of enamel abrading may occur.

For example, referring to the toothbrush 10 illustrated in FIG. 35, the brush head portion 714 moves slightly as the bristles 724 contact an operator's teeth. As more force is applied, the movement of the brush head portion 714 will increase. It is contemplated that as the force reaches the level of a predetermined force, for example, a force of approximately 2N, the movement of the brush head portion 714 will actuate the switch 726 and the light source 716 will be activated. To ensure that the brushing force meets the minimal level desired, the switch 726 may be configured with a spring actuator having a known stiffness. Thus, the switch 726 could be configured such that it is actuated only when a force of at least 2N is applied to the brush head portion 714. Of course, the predetermined force, or minimum required brushing force, can be changed by configuring the switch 726 with a spring actuator having a different stiffness.

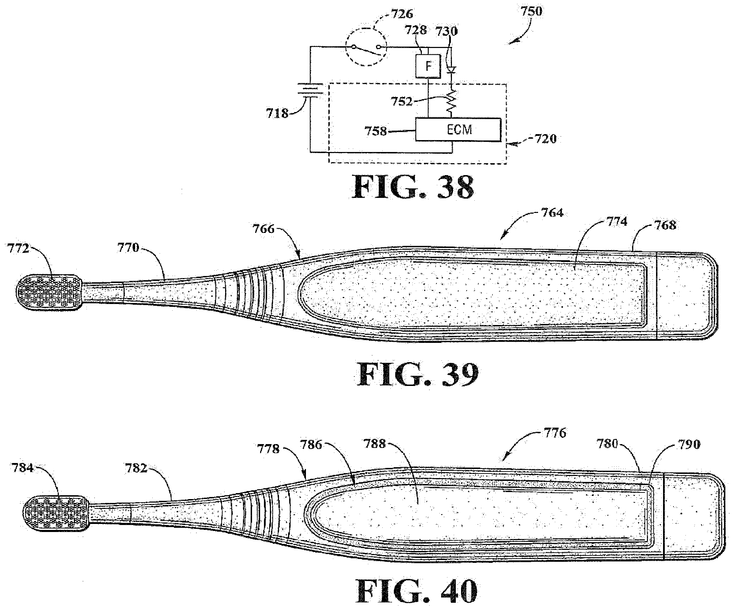

As an alternative to configuring the switch 726 with a spring actuator to control activation of the light source 716, a load cell, or force sensor 728 (illustrated in FIG. 38 and discussed in more detail below), can be included in the toothbrush 710 to ensure that the light source 716 is activated upon application of the predetermined force.

FIGS. 36-37 illustrate one possible configuration for the switch 726. As seen in FIG. 36, the switch 726 comprises first and second contact plates 729, 731, and a magnet 733 having a limiting device 735. In FIG. 36, the switch 726 is in a first position, configured to prevent activation of the light sources 716. With no force being applied to the brush head portion 714, the magnet 733 is at a distance (d) from a magnetic contact 737 disposed on the first contact plate 729. The magnetic contact 37 is separated from the second contact plate 731, such that the two contact plates 729, 731 are not electrically connected.

As a force is applied to the brush head portion 714, the magnet 733 begins to move toward the two contact plates 729, 731, until the attraction from the magnet 733 causes the magnetic contact 737 to move toward the magnet 733. The magnetic contact 737 then makes contact with the second contact plate 731, thereby placing the switch 726 in a second position and activating the light sources 716 (see FIG. 37). The switch 26 can be configured so that the magnetic contact 737 is impelled toward the second contact plate 731, only after the first predetermined force has been applied to the brush head portion 714.

By adjusting various parameters such as the distance between the contact plates 729, 731, the strength of the magnet, and size of the limiting device 735, the predetermined force can be adjusted. Thus, the switch 726 can be configured to require different amounts of force to activate or stop the light sources 716.

The toothbrush body 712 has a generally cylindrical shape, though it could be made in almost any shape desired. For example, the translucent portion 742 could be beveled or faceted to create a prismatic affect as the emitted light passes through it. The minimal space required by the light source 716 and the control circuit 720, allows for design flexibility. Indeed, the present invention contemplates the use of more traditional toothbrush bodies, for example, ones having rectangular cross sections. In addition, the light source need not be an LED, but rather, may be a light bulb. As explained below in conjunction with other embodiments of the invention, the light source is not limited to only one particular kind-e.g., LED.

In addition to varying the arrangement of the light sources, the pattern of light generated by any set of light sources may be varied, depending on the configuration of the control circuit. For example, the toothbrush 710 may be configured with a control circuit that allows the LED to varying in intensity of the light emitted.

FIG. 38 shows a simple wiring schematic of a circuit 750 that can be used in the embodiment illustrated in FIG. 35. The circuit 750 includes the battery 718, the switch 726, the LED 730 and the control circuit 720. The control circuit 720, includes resistors 752 and an electronic control module (ECM) 758. Any suitable ECM may be used with a control circuit such as the control circuit 720, though a Philips 51 LPC is one type of ECM known to work in this application. As discussed above, activation of the light sources in a light emitting toothbrush, such as the toothbrush 710, may be controlled by a switch that includes a spring having a known stiffness. Alternatively, a load cell, such as the force sensor 728 may be used to sense the force being exerted on the brush head portion 714, and provide a brush force input signal to the ECM 758. This allows the ECM 758 to appropriately control the LED 730 based on the brush force input signal. It is readily understood by those skilled in the art that the circuit 50 shown in FIG. 8, represents but one of many circuits that can be used with the present invention. For example, a separate power supply, along with capacitive and additional resistive elements, can be added to the circuit to provide greater control of the power being delivered to the LED.

The embodiments described thus far have each included a switch that is actuated by a force applied to a brush head portion of a toothbrush body, such as the brush head portion 714 shown in FIG. 35. This configuration may be particularly useful when an object of the toothbrush is to train an operator to apply a proper amount of force during brushing. There are however, other ways in which a switch, such as the switch 726, may be actuated. For example, FIG. 39 shows a toothbrush 764 comprising a toothbrush body 766 that includes a handle portion 768 and a brush head portion 770 including bristles 772. The handle portion 768 includes a compressible portion 774 that is configured to be compressed when an operator uses the toothbrush 764. The compressible portion 774 comprises a non-rigid material, such as an elastomer. Alternatively, FIG. 40 shows a toothbrush 776 comprising a toothbrush body 778 including a handle portion 780 and a brush head portion 782, including bristles 784. The handle portion 780 includes a compressible portion 786 that comprises a rigid portion 788 surrounded by a non-rigid portion 790. This configuration may provide a compressible portion having greater stiffness than the compressible portion 774 shown in FIG. 39.

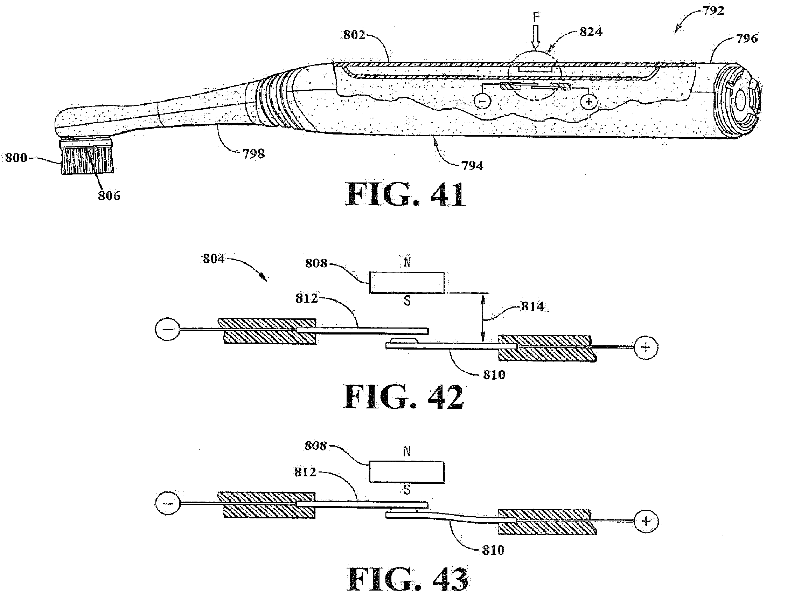

The embodiments shown in FIGS. 39 and 40 have compressible portions 774, 786 disposed on the same side of the toothbrush body as the bristles 772, 784. Of course, a compressible portion of a toothbrush handle portion may be located virtually anywhere on a toothbrush body, for example, on a side of the toothbrush body opposite the bristles. FIG. 41 shows a toothbrush 792 comprising a toothbrush body 794 including a handle portion 796 and a brush head portion 798, having bristles 800. The handle portion 796 includes a compressible portion 802 that is disposed on a side of the toothbrush 792 opposite the bristles 800. A switch 804 is disposed in relation to the compressible portion 802 such that compressing the compressible portion 802 actuates the switch 804. Actuating the switch 804 activates light source 806 which may be an LED as described above, or may be a light bulb. The switch 804 is shown in detail in FIGS. 42 and 43. The switch 804 includes a magnet 808, a magnetic plate 810, and a nonmagnetic plate 812. When a force (F) is exerted on the compressible portion 102, the force causes the magnet 808 to move in close proximity to the magnetic and nonmagnetic plates 810, 812. When the distance between the magnet 808 and the magnetic plate 810 drops below a fixed distance 814, the two plates 810, 812 contact each other (see FIG. 43), thereby activating the light sources 806.

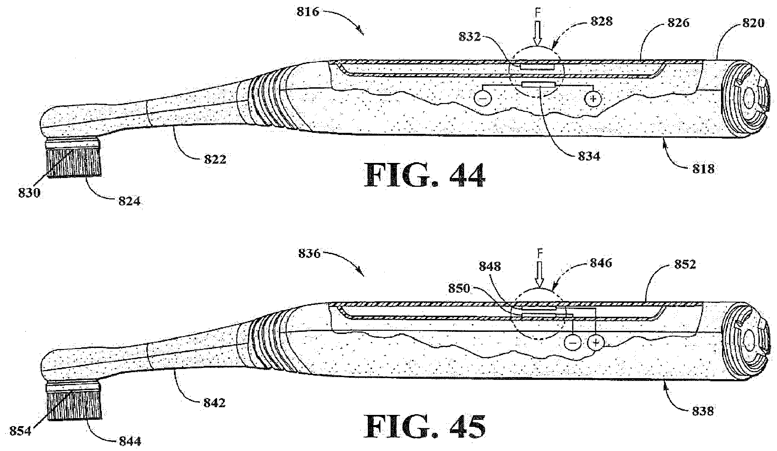

Other types of switches may be used with a toothbrush having a compressible portion, two of which are shown in FIGS. 44 and 45. FIG. 44 shows a toothbrush 816 comprising a toothbrush body 818 including a handle portion 820 and a brush head portion 822, including bristles 824. The handle portion 818 includes a compressible portion 826. A switch 828 is disposed in relation to the compressible portion 826 such that compressing the compressible portion 826 actuates the switch 828, which activates light sources 830. The switch 828 comprises a magnet 832 and a Hall effect sensor 834. The magnet 832 is located beneath the compressible portion 826 such that application of a force (F) to the compressible portion 826 causes the distance between the magnet 832 and the Hall effect sensor 834 to decrease. When this distance is small enough, current flows through the Hall effect sensor 834 and the light sources 830 are activated.

Another type of switch that can be used in conjunction with a compressible portion on a toothbrush handle is shown in FIG. 45. A toothbrush 836 comprises a toothbrush body 838 including a handle portion 840 and a brush head portion 842, including bristles 844. A switch 846 comprises first and second contact plates 848, 850 disposed in relation to a compressible portion 852 of the handle portion 838 such that compressing the compressible portion 852 causes the two contact plates 848, 850 to move closer to each other until they contact, thereby actuating the switch 846 and activating light sources 854.

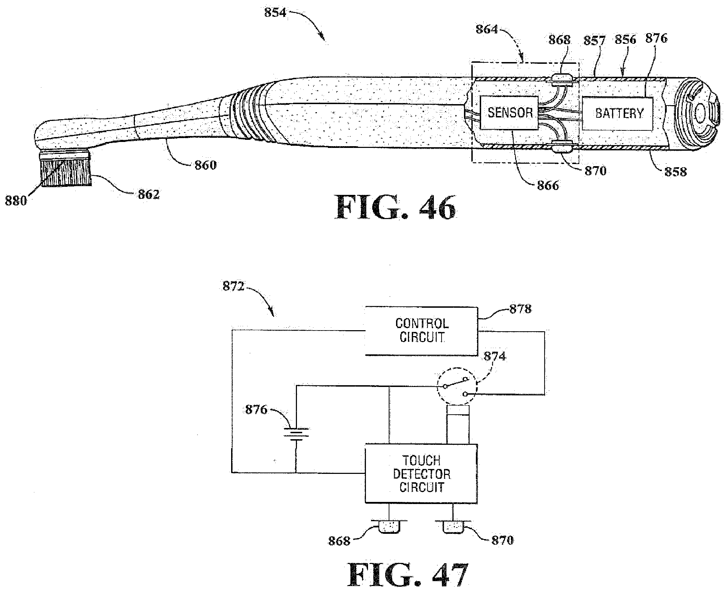

FIG. 46 illustrates another way by which the light source in a toothbrush may be activated. A toothbrush 854 comprises a toothbrush body 856 including a handle portion 858 and a brush head portion 860, including bristles 862. The toothbrush 854 includes a sensing device 864 which comprises a capacitive sensor 866 attached to a pair of tactile sensors 868, 870 partially disposed on an external portion 871 of the toothbrush body 856. The presence of an operator's hand on the tactile sensors 868, 870 closes a switch 874 (see FIG. 47) that allows current to flow from a battery 876 to a control circuit 878 for controlling light source 880. The control circuit 878 may be configured similarly to the control circuit 750 shown in FIG. 38, or may have any configuration suitable to its use in the circuit 872. Thus, the mere presence of an operator's hand on the tactile sensors 868, 870 causes the light source 880 to emit light according to the programming and configuration of the control circuit 878.

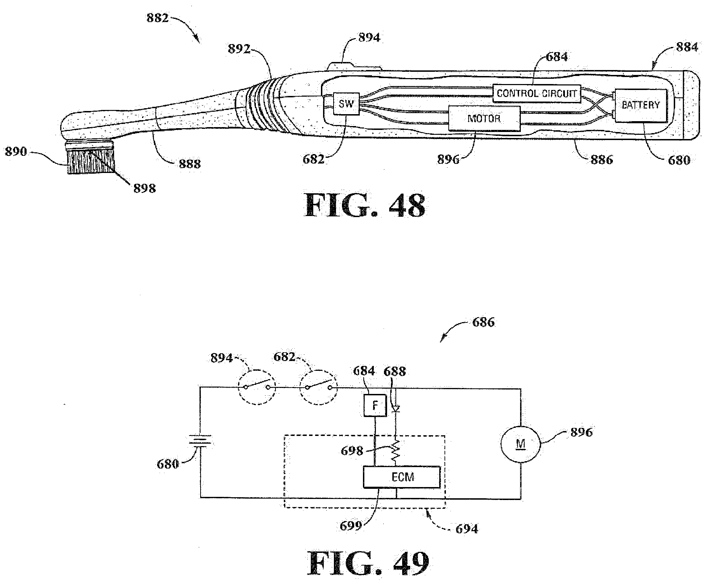

The embodiments thus far described include only manual-i.e., not motorized-toothbrushes. It is important to note that the present invention can be easily utilized with motorized electric toothbrushes as well. FIG. 48 shows a simplified descriptive view of a motorized electric toothbrush 882. The toothbrush 882 comprises a toothbrush body 884 including a handle portion 186 and a brush head portion 888. The brush head portion 888 includes a bristle head 890. A flexible portion 892 is provided that facilitates some movement of the brush head portion 888 when a force is applied to it. A first switch 894 is disposed on the handle portion 886, and is configured to connect a motor 896 and light source 898 to an electric source, such as battery 680. When engaged, the motor 896 drives the bristle head 890. The first switch 892 has a first position for preventing activation of the light source 898 and the motor 896, and a second position for facilitating automatic activation of the light source 898 and the motor 896.

A second switch 682 is disposed within the toothbrush body 884. The second switch 682 has a first position for preventing activation of the light sources 898 and the motor 896, and a second position for activating the light sources 898 and the motor 896 when the first switch is in the second position. The second switch 682 is placed in the second position when a predetermined force is applied to the brush head portion 888. The force may be applied during use, when the bristle head 890 is brought into contact with a user's teeth.

As in the previous embodiments, the predetermined force may be set by using a spring having a known stiffness. Specifically, such a spring may be used to resist a force applied to the brush head portion 888. In this way, the spring force will need to be at least partially overcome-i.e., a force equal to the predetermined force will need to be applied to the brush head portion 888-in order to place the second switch 682 in the second position.

As an alternative to using a spring to control the predetermined force, a separate load cell, or force sensor 684 may be utilized (see FIG. 49). FIG. 49 shows a simple wiring schematic of a circuit 686 that can be used with a motorized toothbrush 882. As shown in FIG. 19, the light source 898 comprises LED 688 and is controlled by electrical circuitry, or a control circuit 694. The control circuit 694 includes resistor 698 and an electronic control module (ECM) 699.

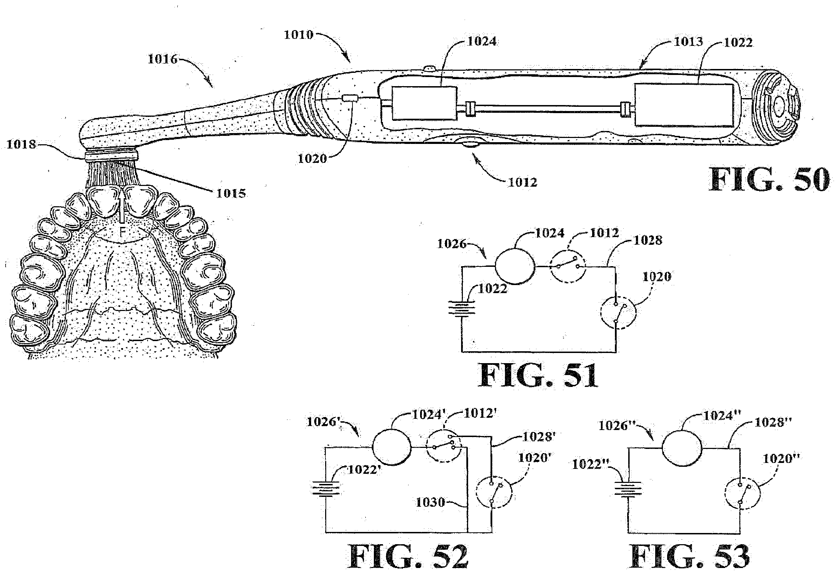

Another useable pressure sensor is disclosed in US2003/0135940 to Lev et al. FIG. 50 shows a simplified descriptive side view of a motorized electric toothbrush 1010. A first switch 1012, located in a handle portion 1013, has a first or "off position, and a second or "automatic" position, which places the toothbrush 1010 in an automatic mode. While the toothbrush 1010 is in the automatic mode, a LED 1015 is engaged only when a force (F) is exerted on a removable head portion 1016. This occurs when a bristle head 1018 sufficiently contacts an operator's teeth. As used here and throughout, the term "sufficiently contacts" implies a contact that is sufficient to cause a slight movement of at least a part of the removable head portion 1016 in the direction of the force. The force exerted by an operator (a user of the toothbrush) during normal brushing typically constitutes a sufficient contact. Thus, as the user begins brushing, a second switch 1020 automatically moves from a first position to a second position, an electric circuit is completed, and current flows from a battery 1022 to the LED driver 1024 that regulates and transmits power to LED 1015.

FIG. 51 shows a simple wiring schematic 1026 of a circuit for the toothbrush 1010 shown in FIG. 50. The LED driver 1024 is electrically connected between an electric source (the battery 1022) and the first switch 1012. When the first switch 1012 is in the first, or "off' position, the circuit 1028 is open and there is no voltage across LED driver 1024. When the first switch 1012 is in the second, or "automatic" position, control of the current flow to LED driver 1024 is transferred to the second switch 1020. While the toothbrush 1010 is in the automatic mode, the LED driver 1024 is only engaged when a force (such as (F) shown in FIG. 1) is applied to the bristle head 1018. An exception to this occurs when the toothbrush is programmed with a "delayed off feature, discussed in more detail below. With the delayed off feature, the LED 1015 continues to operate for a short time after the force is removed from the bristle head 1018.

A number of alternative electrical circuits can be used with the toothbrush, two of which are shown in FIGS. 52 and 53. In FIG. 52, a wiring schematic 1026' shows a LED driver 1024' wired between a battery 1022' and a first switch 1012'. The first switch 1012' is a three position switch, having a first position in which circuits 1028', 1030 are both open. While the first switch 1012' is in the first position, no current can flow to the LED driver 1024', and the LED 1015 remains off. The first switch 1012' has a second position in which control of the current flow to LED driver 1024' is transferred to a second switch 1020'. When the second switch 1020' is in a first position, the circuit 1028' is open, and LED 1015 remains off. When a force is applied to a bristle head of the toothbrush, the second switch 1020' automatically moves to a second position such that the circuit 1028' is closed and the LED driver 1024' is engaged. The first switch 1012' also has a third position, in which the circuit 1030 is closed, and the toothbrush operates continuously.

Yet another wiring configuration is illustrated in the wiring schematic 1026" shown in FIG. 53. In this configuration, there is only one switch 1020" to control the flow of current from a battery 1022" to a LED driver 1024". When the switch 1020" is in a first position, circuit 1028" is open, thereby preventing the LED 1015 from turning on. When the switch 1020" is in a second position, the circuit 1028" is closed and current flows to LED driver 1024". As in the previous wiring configurations, the switch 1020" automatically moves from the first position to the second position when a force is applied to a bristle head of the toothbrush. As described above, each of the switches 1020, 1020', and 1020" "automatically" moves from a first position to a second position when the toothbrush is used by an operator. This implies that the user need not manually place the switch in the second position. Rather, the contact between the user's teeth and the bristle head automatically places the switch 1020, 1020', or 1020" in the second position.

Toothbrushes in accordance with these embodiments can be configured such that applying a force to the handle portion, rather than the bristle head, effects operation of the LED 1015 when it is in the automatic mode. For example, any of the switches 1020, 1020', 1020" can be positioned within the handle portion 1013 of the toothbrush 1010, shown in FIG. 1. In such a case, the switch may be automatically moved from the first position to the second position not by a force applied to the bristle head 1018, but rather, by a force applied to some part of the handle portion 1013. Embodiments utilizing this feature are described in more detail below.

Each of the wiring configurations shown in FIGS. 51-53 allow a toothbrush to be used in an automatic mode. That is, the toothbrush motor is engaged whenever a force is applied to that portion of the toothbrush that contains the switch 1020, 1020', or 1020". This facilitates ease of use, eliminating the need to operate a typical button switch after the bristle head is placed in the user's mouth. Another advantage of such a configuration is that a consumer can engage the toothbrush motor while the toothbrush is still packaged-i.e., prior to sale. In this way, the consumer can evaluate the operation of the toothbrush before purchase. Some prior art toothbrushes have a multifunction switch configured such that the consumer operates the toothbrush in the package using one activation mode, then operates the toothbrush during normal use in another activation mode. Such is not the case with the present disclosure, which affords the consumer the opportunity to activate the toothbrush in the package substantially as it will be activated during normal use. In today's consumer savvy environment, this feature provides another advantage over prior art toothbrushes.

FIG. 52 shows an exploded view of the electric toothbrush 1010 in accordance with another embodiment. The toothbrush 1010 includes the handle portion 1013 and the removable head portion 1016, which is shown having first and second housing elements 1032, 1034. The handle portion 1013 will usually be made from a polymeric material, and may be opaque, clear, or translucent. When the handle portion 1013 is clear or translucent, the toothbrush operator may see the movement of some of the toothbrush components when the motor 1014 is engaged. In addition, aesthetically pleasing features such as flashing lights (not shown) can be added to the components within the handle portion 1013 to augment the visual appearance. The removable head portion 1016 also includes a shaft 1036 that on one end has a pinion carrier 1038 and on the other a yoke 1040 configured to attach to a drive shaft 1042. A pinion 1044 is attached to the bristle head 1018 with a threaded fastener 1046 and a washer 1048. The pinion 1044 interfaces with a rack 1050, only a portion of which is visible through an opening 1052 in the first housing element 1032. Also included in the removable head portion 1016 is a snap ring 1054 that is manufactured in different colors such that removable brush heads belonging to different users can have different colored snap rings for easy identification.

This embodiment includes a rocker element 1056, which serves a number of functions. First, it contains clips 1058 (only one of which is visible in this view) that help secure the removable head portion 1016 to the handle portion 1013. In addition, trunnions 1060 (only one of which is visible), rotate in apertures 1062 thereby allowing the rocker element 1056 to pivot as force is applied to the removable head portion 1016. As the rocker element 1056 pivots about the trunnions 1060, a pin 1064 moves within a slot 1066. The slot 1066 is located in a first casing portion 1068 which also contains one of the apertures 1062 in which one of the trunnions 1060 rotates. Also located in the first casing portion 1068 is the first switch 1020, which comprises first and second contact plates 1070, 1072. As noted above, the first switch 1020 is optional (see FIG. 53), in which case, the toothbrush 1010 will always be in the automatic mode.

The contact plates 1070, 1072 are attached to the first casing portion 1068 in such a way that movement of the pin 1064 within the slot 1066 selectively causes the contact plates 1070, 1072 to contact each other and electrically connect. Electrically connecting the contact plates 1070, 1072 places the second switch 1020 is in the second position. This means that when the toothbrush 1010 is in the automatic mode of operation-i.e., when the first switch 1012 is in the second position-electrical connection of the contact plates 1070, 1072 engages the LED driver 1024 and causes LED 1015 to turn on. Thus, when the toothbrush 1010 is in the automatic mode of operation, sufficient contact of the bristle head 1018 with the user's teeth will cause a slight deflection of the removable head portion 1016. This in turn causes the rocker element 1056 to pivot on its trunnions 1060, thereby moving the pin 1064 within the slot 1066. When the pin 1064 causes electrical connection of the contact plates 1070, 1072, the LED driver 1024 is engaged without the user having to manually actuate any switches. Hence, turning on of LED 1015 is "automatic". The contact plate 1070 also acts like a spring, so that when the bristle head 1018 is not in contact with the user's teeth, the contact plate 1070 pushes against the pin 1064 and biases away from the contact plate 1072. Thus, the second switch 1020 returns to the first position when the bristle head 1018 is no longer in contact with the user's teeth.

Although the second switch 1020 returns to the first position when the bristle head 1018 is no longer in contact with the user's teeth, the LED driver 1024 may not immediately disengage. The action of the LED driver 1024 in this situation is dependent upon the configuration of a printed circuit (PC) board 1074. The PC board 1074 is an electronic controller that controls the electrical components of the toothbrush 1010. The PC board 1074 can be configured such that the LED driver 1024 continues to operate for a finite time after the second switch 1020 is moved from the second position to the first position. The finite time can be a very short interval, perhaps as little as a fraction of a second. This feature may be useful when the bristle head 1018 momentarily disengages contact with the user's teeth during normal brushing. During the short interval, until the time the bristle head 1018 is again in contact with the user's teeth, the LED driver 1024 will continue to run.

Although the wires are removed from this figure for clarity, the simple wiring is easily understood by one skilled in the art. The PC board 1074 is wired to the motor 1014 at terminals 1076, 1078. Similarly, battery terminals 1080, 1082 are wired to the PC board 1074 through spring terminals 1084, 1086. The PC board 1074 can also be configured to control other functions in addition to the "delayed off' feature. For example, the PC board 1074 may not only control the delay of turning on/off LED 1015, but also the intensity of LED 1015 output. In addition, if indicator lights are used in conjunction with a transparent or translucent cover, as described above, the PC board 1074 can be configured to control the colors, duration, and sequence of such lights. In addition, the PC board 1074 can be configured to control sound elements, either alone, or in combination with the LED or indicator lights.

The first switch 1012 includes a switch cover 1088 and a switch button 1090. When an operator presses the switch cover 1088 the switch button 1090 contacts an electrical component 1092 of the PC board 1074, thereby placing the switch 1012 in the second position. Further pressing of the switch cover 1088 toggles the switch 1012 between the first and second positions. The handle portion 1013 also includes a drive shaft seal 1094 and a seal support 1096. The drive shaft seal 1094 helps to ensure that fluid does not reach the electrical components of the toothbrush 1010. The PC board 1074 includes an indicator LED 1098 that is visible to a user through a translucent cover 1100. The indicator LED 1098 may be used to indicate when the first switch 1012 is in the second position-i.e., when the toothbrush 1010 is in the automatic mode-or may be used to indicate when the battery 1022 is being charged. The battery 1022 is held in place by an end cap 1102 that is provided with an O-ring seal 1104 to further ensure that fluids do not reach the electrical components of the toothbrush 1010. Also included in the handle portion 1013 is a seat element 1106 that allows the toothbrush 1010 to be laid on a flat surface such that the bristle head 1018 remains pointing upward. This helps to keep the toothbrush 1010 stationary on a surface that is not level, and keeps the bristle head 1018 from contacting the surface. Aesthetic features 1108 are added to enhance the visual appeal of the toothbrush 1010.

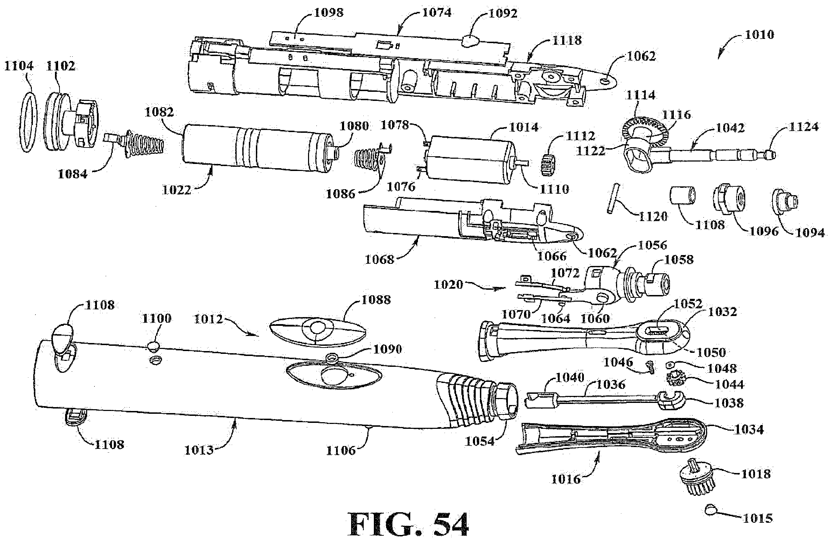

The sensors described in these embodiments may be used in any type of dental hygiene implement. In the embodiments shown in FIGS. 54, 58-60, the dental hygiene implement is a motorized toothbrush. The reciprocating movement of the drive shaft 1042 is guided by a bushing 1108. The actual movement of the drive shaft 1042 resembles a typical slider crank mechanism. The motor 1014 has a rotating motor shaft 1110 that has a spur gear 1112 attached to it. The spur gear 1112 intermeshes with and rotates a ring gear 1114 that has integrally attached to it a cam 1116. The ring gear 1114 and the cam 1116 are held in a second casing portion 1118 with a pin 1120. The cam 1116 rotates within a cam follower 1122 that is attached to the drive shaft 1042. Thus, the rotational motion of the motor shaft 1110 is translated into reciprocating motion of the drive shaft 1042. When the removable head portion 1016 is attached to the handle portion 1013, the yoke 1040 connects to a head 1124 on the drive shaft 1042 such that the shaft 1036 reciprocates along with the drive shaft 1042. This in turn moves the pinion 1044 along the rack 1050 which causes the bristle head 1018 to translate and rotate simultaneously.

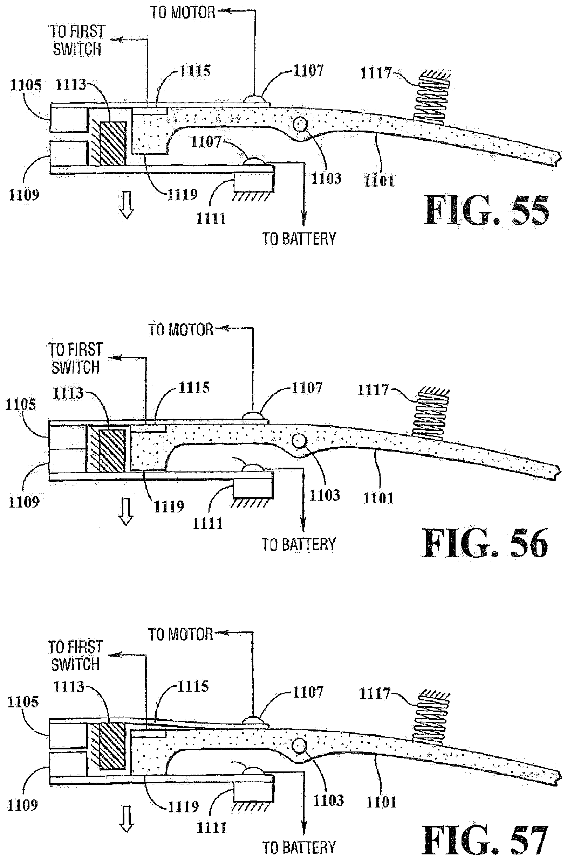

An alternative configuration for the second switch 1020 is shown in FIGS. 55-57. This configuration provides for automatically stopping operation of LED 1015 on the toothbrush when the force on the bristle head in response to contact with the operator's teeth exceeds a predetermined level. FIG. 55 shows a portion of a rocker element 1101 that pivots about trunnions 1103 (only one of which is visible). A first contact plate 1105 is attached to the rocker element 1101 with a fastener 1107. A second contact plate 1109 is attached to a casing with another fastener 1107, a portion of the casing being shown as 1111. The casing has a stop block 1113 integrally formed therewith. The rocker element 1101 includes an electrically conductive contact pad 1115, which is an option that can be used when the first switch is a three-position switch (see FIG. 51). As illustrated in FIG. 55, the contact pad 1115 is electrically connected to the contact plate 1105, and when a three-position first switch is used, the contact pad 1115 will be wired to the first switch. Thus, when the first switch is in the third position, the toothbrush motor (such as 1014 shown in FIG. 51) will operate continuously. A spring 1117 is disposed between the rocker element 1101 and another portion of the casing (not shown), and biases the first contact plate 1105 away from the second contact plate 1109.

FIG. 56 is illustrative of automatic operation of the toothbrush. As the bristle head contacts the operator's teeth, the rocker element 1101 pivots about the trunnions 1103, thereby electrically connecting the contact plates 1105, 1109. As illustrated in FIGS. 55-57, the first contact plate 1105 is wired to the LED driver, and the second contact plate 1109 is wired to the battery. Thus, when the first switch is in the second position, or when there is only one switch (see FIG. 52), the electrical connection of the contact plates 1105, 1109 causes operation of LED 1015 on the toothbrush. If the operator continues to apply force to the bristle head beyond a predetermined level, the first contact plate 1105 will impinge on stop block 1113, but the rocker element 1101 will continue to pivot (see FIG. 57). A protrusion 1119 on the rocker element 1101 then contacts the second contact plate 1109 and pushes it away from the first contact plate 1105. This opens an electric circuit and turns off the LED 1015. Even if the toothbrush has a "continuous on" feature, the LED 1015 will still turn off when the first contact plate 1105 impinges on the stop block 1113. This is because the contact pad 1115 will no longer be in contact with the first contact plate 1105. The predetermined level at which the LED 1015 turns off can be easily adjusted by changing the spring 1117, the size of the stop block 1113, or the size of the protrusion 1119.

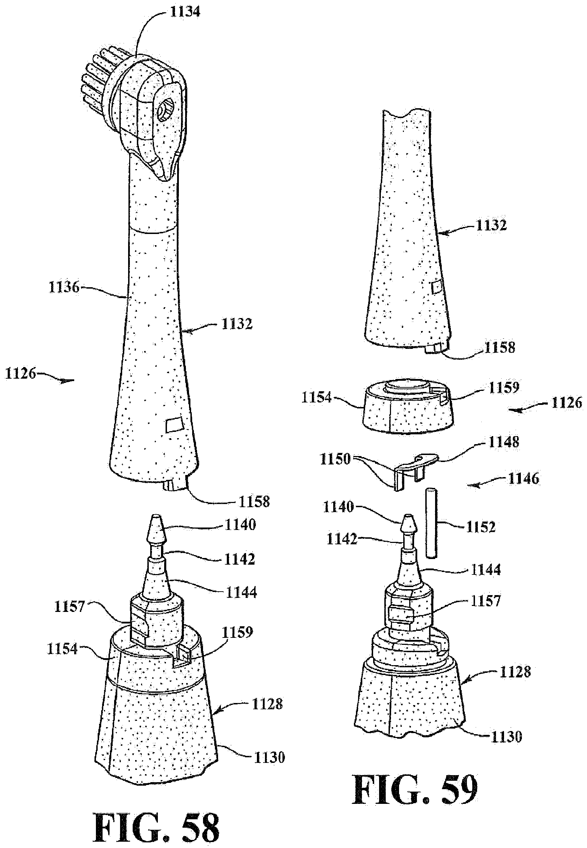

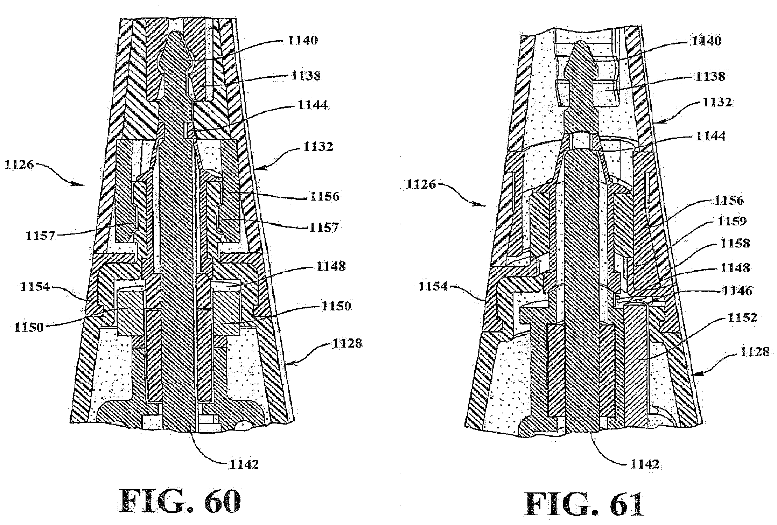

FIGS. 58-62 show portions of a toothbrush 1126 in accordance with another embodiment. The toothbrush 1126 comprises a handle portion 1128 that includes a first housing 1130, and a removable head portion 1132 that includes a bristle head 1134 and a second housing 1136. This includes a shaft and a pinion which interfaces with a rack to drive the bristle head 1134. A yoke 1138, seen in FIGS. 60 and 61, connects to a head 1140 of a drive shaft 1142 which reciprocates when a motor (not shown) is engaged. A seal 1144 is disposed around the drive shaft 1142 to protect the electrical components of the toothbrush 1126 from contamination by fluids.

As in the previous embodiment, the toothbrush 1126 includes an automatic mode of operation. To facilitate the automatic mode of operation, the toothbrush 1126 has a first switch (not shown) that is configured as in the previous embodiment. A second switch 1146, seen in FIGS. 59 and 61, includes a contact plate 1148 having legs 1150 and a contact rod 1152. The contact plate 1148 and the contact rod 1152 are disposed within the handle portion 1128 and are covered by a seal 1154. Similar to the contact plates 1070, 1072 used in the first embodiment, the contact plate 1148 and the contact rod 1152 are wired to a PC board (not shown).

The method by which the removable head portion 1132 attaches to the handle portion 1128 is also different from the first embodiment. An adaptor 1156, seen in FIGS. 60 and 61, is located inside the housing 1136 of the removable head portion 1132, and snaps into recesses 1157 in the handle portion 1128, (see FIG. 60). This attachment allows the removable head portion 1132 to be securely attached to the handle portion 1128, and at the same time allows the head portion 1132 to pivot in relation to the handle portion 1128 when the bristle head 1134 sufficiently contacts the user's teeth. As consistently used throughout the various embodiments, "sufficiently contacts" merely implies a contact that is sufficient to cause a slight movement of at least a part of the removable head portion 1132.

As the removable head portion 1132 undergoes the slight pivoting motion caused by contact with the user's teeth, a projection 1158 pushes into a notch 1159 in the seal 1154. As the projection 1158 moves into the notch 1159, it pushes the seal 1154 against the contact plate 1148. With the legs 1150 held stationary, the contact plate 1148 deflects in a spring-like fashion until it contacts the contact rod 1152. This places the second switch 1146 in the second position, and enables LED 1015 to turn on when it is in the automatic mode. The spring-like deflection of the contact plate 1148 also acts to bias it away from the contact rod 1152, to turn off LED 1015 when the bristle head is not in contact with the user's teeth. As in the previous embodiment, the PC board can be configured such that the LED 1015 does not disengage immediately, but rather, remains engaged for a short time after the bristle head is removed from the user's teeth.

Although both of the embodiments described above have a two-position first switch, as illustrated schematically in FIG. 51, a three-position switch (as shown in FIG. 52) can be used. Alternatively, the first switch can be eliminated, as in FIG. 53, so that the toothbrush is always in the automatic mode. The two toothbrushes described above include removable brush head portions; however, either can be made with a non-removable brush head portion. In fact, any of the embodiments described herein can be made with a non-removable brush head portion, which may be particularly well suited to disposable toothbrush designs.

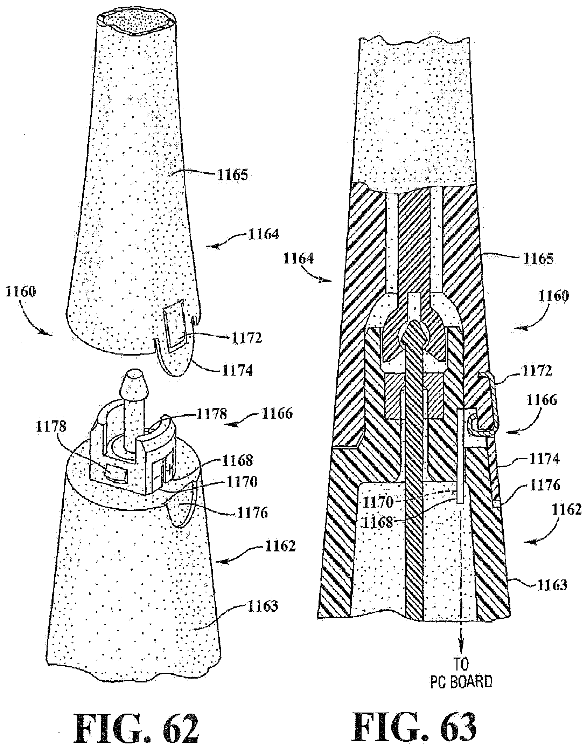

Portions of a third embodiment are shown in FIGS. 62 and 63. In this embodiment, a toothbrush 1160 includes a handle portion 1162 that has a first housing 1163, and a removable head portion 1164 that has a second housing 1165 and a bristle head (not shown). As in the previous embodiments, the toothbrush 1160 includes a first switch (not shown) having a first, or "off' position that prevents the LED 1015 from turning on, and a second, or "automatic" position that allows the LED 1015 to function in an automatic mode. A second switch 1166 comprises first and second stationary contact plates 1168, 1170, and a third contact plate 1172. The removable head portion 1164 includes a projection 1174 that fits into a notch 1176 in the first housing 1163 of the handle portion 1162.

The removable head portion 1164 attaches to the handle portion 1162 at snaps 1178. This connection allows the removable head portion 1164 be securely attached to the handle portion 1162, and at the same time allows the head portion 1164 to pivot in relation to the handle portion 1162 when a bristle head (not shown) sufficiently contacts the user's teeth. As the removable head portion 1164 pivots, the third contact plate 1172 contacts, and thereby electrically connects, the stationary contact plates 1168, 1170. This places the second switch 1166 in the second position, and causes LED 1015 to turn on when it is in the automatic mode. The projection 1174 also acts as a spring as the removable head portion 1164 pivots, thereby keeping the third plate 1172 biased away from the stationary plates 1168, 1170 when the bristle head is not in contact with the user's teeth.

In each of the embodiments described above, the second switch was automatically moved from the first position to the second position when a force was applied to the bristle head. Specifically, a force on the bristle head in response to its contact with the operator's teeth caused the second switch to move to the second position and the motor was engaged. As previously noted however, the second switch need not be activated by a force on the bristle head. Rather, the second, switch may be located such that it is automatically placed in the second position when the user grips the handle portion. One way to accomplish this is to provide the handle portion with a compressible portion, and dispose the second switch in relation to the compressible portion such that compressing the compressible portion moves the second switch from the first position to the second position, thereby turning on LED 1015.

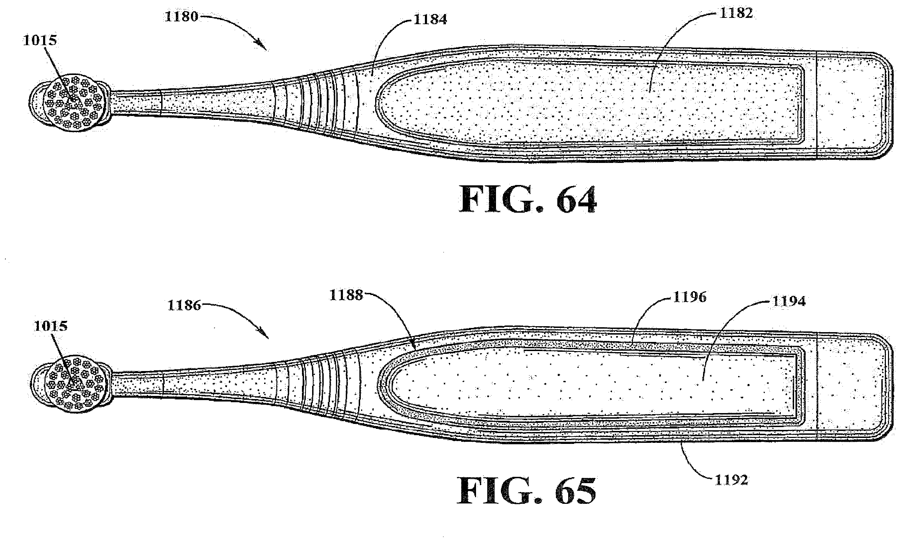

FIGS. 64 and 65 illustrate two embodiments of toothbrushes having differently configured handle portions. FIG. 64 illustrates a toothbrush 1180 having a compressible portion 1182. The compressible portion 1182 can be molded integrally with toothbrush handle housing 1184, or may be attached in a separate operation. The compressible portion 1182 is typically made from a polymeric material that deflects when the toothbrush is used in a normal brushing operation. The handle housing 1184 may be configured with a relatively small, or a relatively large compressible portion. In this embodiment, the compressible portion 1182 occupies a large area of the handle housing 1184, thereby helping to ensure that users having different gripping preferences will be accommodated.

FIG. 65 illustrates a toothbrush 1186 having a compressible portion 1188 located in a housing 1190 of a handle portion 1192. In this embodiment, the compressible portion 1188 includes a rigid portion 1194 and a non-rigid portion 1196. When a user compresses the compressible portion 1188, the non-rigid portion deflects, thereby moving a switch (not shown) from a first position to a second position to turn on LED 1015. Having a two-piece compressible portion such as 1188 not only changes the look, but also the feel of the toothbrush when compared to a toothbrush having a single piece compressible portion. Thus, the designer is allowed flexibility with regard to both form and function.

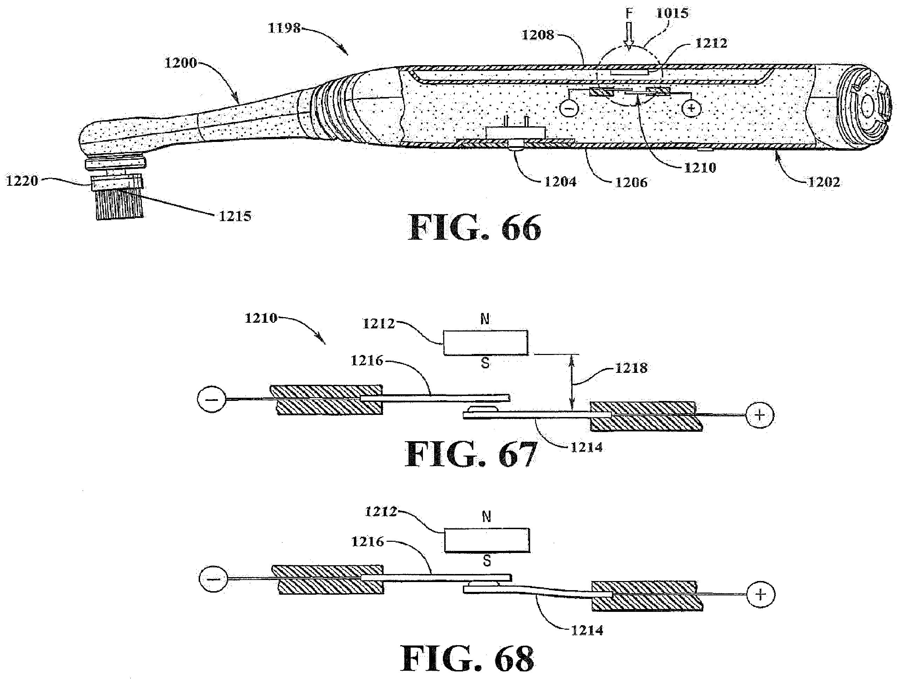

FIG. 66 shows a toothbrush 1198 that includes a removable head portion 1200 and a handle portion 1202. The handle portion 1202 includes a first switch 1204 which has first and second positions for respectively turning off and on LED 1015. The handle portion 1202 includes a handle housing 1206 that has a compressible portion 1208. Disposed within the handle portion 1202 in close proximity to the compressible portion 1208, is a second switch 1210, shown in detail in FIG. 67.

The switch 1210 shown in FIG. 67 includes a magnet 1212, a magnetic plate 1214, and a non-magnetic plate 1216. When the first switch 1204 is in the second position, motorized operation of the toothbrush 1198 occurs only when a force (F) is exerted on the compressible portion 1208. This force causes the magnet 1212 to move in close proximity to the magnetic and non-magnetic plates 1214, 1216. When the distance between the magnet 1212 and the magnetic plate 1214 drops below a fixed distance 1218, the two plates 1214, 1216 contact each other (see FIG. 68). When the first switch 1204 is in the second position, and the two plates 1214, 1216 contact each other, the LED driver is engaged (not shown), thereby causing LED 1215 to turn on.

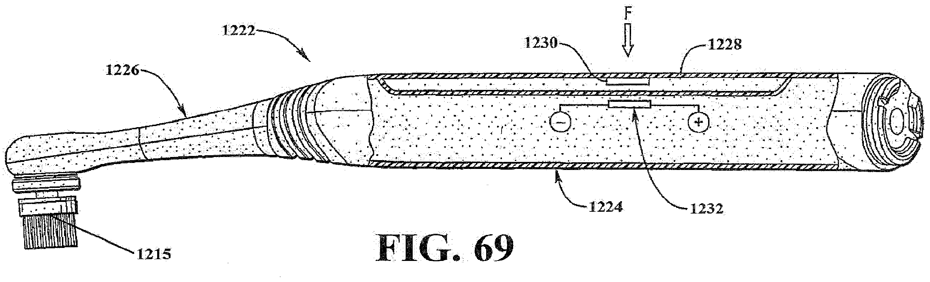

FIG. 69 shows another embodiment of a toothbrush 1222. The toothbrush 1222 has a handle portion 1224 and a removable head portion 1226. The handle portion 1224 includes a compressible portion 1228. In this embodiment, toothbrush 1222 only has one switch 1229 which comprises a magnet 1230 and a Hall effect sensor 1232. The magnet 1230 is located beneath the compressible portion 1228, and application of force (F) to the compressible portion 1228 causes the distance between the magnet 1230 and the Hall effect sensor 1232 to decrease. When this distance is small enough, current flows through the Hall effect sensor 1232, and LED 1215 turns on.

In another embodiment the sensor is a moisture detection - uses a moisture sensor to detect a highly moist environment such as the mouth. This can be accomplished through various types of moisture sensors for example; capacitive or chilled mirror dew point sensors. Such a sensor is disclosed in US 2009/0087813 to Cai et al.

These motion sensor embodiments relate to a bio-active oral care instrument, having the ability to active an LED automatically, when the instrument or a portion thereof is exposed to one or more conditions, such as the ambient electrical conductivity, existing in the oral environment. Other conditions and combinations of conditions, such as pH, temperature, solute concentrations, etc. could likewise be detected and used as the basis for automatic operation. Furthermore, aspects of these embodiments are illustrated in the remainder of this disclosure with reference to an electric motorized toothbrush, although it is understood that the operation of any number of oral care instruments, together with the associated advantageous features and/or beneficial effects described herein, could likewise be achieved. Other oral care instruments, for example, include those used in dental drilling, polishing, and grinding; oral suction instruments, oral surgical instruments; and other instruments used in the oral cavity which are powered by motorized devices and especially electrical devices.

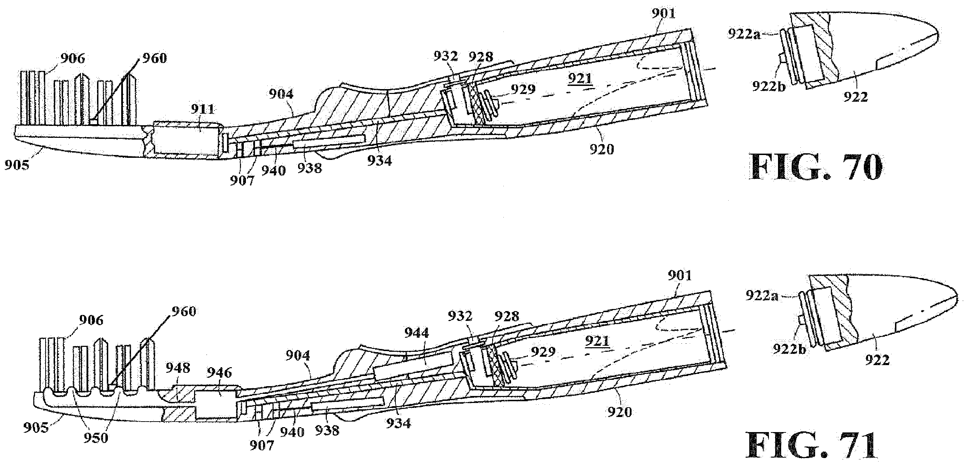

The representative toothbrush illustrated in FIG. 70 has a handle 901 and a head 905 carrying one or more cleaning elements, which are depicted in FIG. 70 as a plurality of bristles 906. Also illustrated is a neck 904 located between, and connecting, handle 901 and head 905. The bristles 906, as shown, form clusters that are anchored to the head 905 and provide a profiled brushing surface with their free ends. Other bristle configurations are of course possible, as well as removable/exchangeable bristle clusters. Different types of cleaning elements (e.g., elastomeric wipers, nodules, pointed structures, etc.) may also be carried on head 905 instead of, or in addition to, bristles.

The neck 904 is provided with electrical conducting elements 907 (e.g., an anode and a cathode) that are exposed to the exterior surface of the toothbrush. In other embodiments, the electrical conducting elements 907 can be located on the head 905, for example on the surface opposite that which carries bristles 906. The use of electrical conducting elements 907 on different parts of the toothbrush is also possible. A plurality of electrical conducting elements 907 can also be incorporated in various positions to activate the instrument in the event that sufficient electrical conductivity is established between any given pair(s) of electrical conducting elements 907 located at any desired position. Integrated in the region of the neck 904 which is adjacent to the head 905 is a LED driver 911 that is operably connected to LED 960. The motorized device 911 is operably connected, via electrical connections 934 in the neck 904 to a power source (e.g., a battery, not shown), which may be accommodated in the handle 901. Operably connected and operably connectable refer to the ability of the electrical connections, or other elements, to readily form an electrical circuit (e.g., when a switch is depressed or when a power source is connected or installed). Operably connected and operably connectable may also refer to the ability of mechanical components to be connected to one another in such a manner as to allow or provide for physical movement of one or more elements. The LED driver may be alternatively incorporated in the head 905 or handle 901 of the toothbrush. In representative embodiments, electrical connections 934 may be metal wire or electrically conductive plastic tracks.

In particular embodiments where the toothbrush uses a vibratory device, it will have a vibratory element which can be in the form of an eccentric, which produces mechanical vibrations and can be rotated about an axis located in the longitudinal direction of the toothbrush. Alternatively, instead of an eccentric which can be driven in rotation, it would also be possible to have a vibratory element which can be driven in a translational manner. Otherwise, the bristle-carrying head 905 can be arranged such that it can be moved in relation to the neck 904 in order for the latter, in the case of vibrations produced by motorized device 911, to move in relation to the rest of the toothbrush.

As shown, also accommodated in the handle 901 is a sheath or sleeve 920 which extends in the longitudinal direction of the handle 901 and is made of electrically conductive material. In the representative embodiment shown, both the handle 901 and the sleeve 920 are open to the rear, thus forming a cavity 921 which can be closed from the rear by a closure part 922 and into which it is possible to insert a battery, such as a commercially available, non-rechargeable cylindrical battery, with a defined voltage (e.g. 1.5 V), as the power or voltage source for LED driver 911. It would also be possible, however, for a button cell or for a rechargeable storage batter to be used as the power source. An external power source such as a conventional electrical outlet or a combination of voltage sources may be employed as the power source.

Also shown in the particular illustrative embodiment of FIG. 70 is a spring contact 929 for a positive pole of a battery (not shown), which is fitted in the sleeve 920, on a transverse wall 92S, and is electrically connected to the LED driver 911 through the electrical connections 934 and switch 932, which is installed in the sleeve 920 and can be actuated from the outside of the handle 901. Switch 932 may also be, for example, a magnetic switch pulse switch or a pulse switch arranged on a printed circuit board with further electronic components that store the switching state. In other embodiments, closure part 922 can itself act as a switch, such that electrical contact between the power source and LED driver 911 is established or interrupted by turning closure part 922 to alter the position of contact surface 922b relative to the negative pole of a battery.

It is to be appreciated, as discussed in greater detail below, that switch 932 is not necessary due to the ability of the toothbrush to turn on automatically when in the user's mouth. In some embodiments, therefore, the toothbrush can be "switchless" or "buttonless."

Switch 932 may be depressed or adjusted by the user to effect a number of operating modes. For example, in "on" and "off positions or settings, electrical communication or an electrical circuit between the power source and LED driver 911 may be continually established or continually interrupted, respectively. In the former case, for example, the electrical conducting elements 907 may be bypassed to allow continuous operation of motorized device 911, regardless of the presence of a conductive medium between electrical conducting elements 907. Switch 932 may also have a position corresponding to conditional completion of the electrical circuit.

Also as shown in FIG. 70, the closure part 922 is provided with a threaded stub 922a made of an electrically conductive material, which may be the same material (e.g., a metal such as copper or a conductive plastic) used for the electrical conducting elements 907, electrical connections 934, spring contact 929, and/or sleeve 920. Closure part 922 can be screwed into the handle 901 and/or into the sleeve 920 by way of said threaded stub 922a. The threaded stub 922a is provided with a contact surface 922b which, with the closure part 922 screwed in, comes into abutment against the negative pole of a battery (not shown) when inserted into the sleeve 920. During operation of the motorized toothbrush, this negative pole is electrically connected to LED driver 911 via the threaded stub 922a, the sleeve 920 itself, and electrical connections 934 connecting sleeve 920 to LED driver 911. It would also be possible, instead of through the use of sleeve 920, for the power from the negative pole to be transmitted in some other way, for example using wires or electrically conductive plastic tracks. Instead of the rear closure part 922 being screwed to the handle 901, it would, of course, also be possible to have some other type of releasable connection (e.g. plug-in connection, bayonet connection, etc.) and a corresponding configuration of the contact part interacting with the negative pole of the battery.

One representative characteristic of the oral environment which differs significantly from the surrounding or ambient "non-use" environment is electrical conductivity, which increases directionally with the concentration of electrolytes in the surrounding medium (e.g., saliva). In some embodiments, this "non-use" environment may even include rinsing or submersing the portion of the instrument that is normally placed in the mouth (e.g., the head 905 of the toothbrush) in water (e.g., for pre-wetting or rinsing purposes), since the electrical conductivity of saliva is higher than that of water. This difference can thus be utilized to allow the instrument to "detect" when it is being used and thereby operate in an automatic mode.

Additionally, the combination of water, saliva, and dentifrice (e.g., toothpaste or other ingredient that is generated in the mouth during use of the instrument) often affords even a significantly higher electrical conductivity than saliva alone. This is due to the generation of ions, often in large concentrations, from typical oral care products, including tooth fluoridating, whitening, and/or remineralization products which contain or form aqueous cations, such as sodium (Na⁺), potassium (K⁺), calcium (Ca⁺²), magnesium (Mg⁺²), iron (Fe⁺³), etc. and anions, such as phosphate (PO₄⁻³), diphosphate (P₂O₇⁴), carbonate (CO₃⁻²), fluoride (F⁻), chloride (Cl⁻), etc.

In view of the above, the increase in electrical conductivity surrounding a portion of the toothbrush, e.g., head 905 or head 905 and neck 904, when placed in the mouth, can be used to complete an electrical circuit, together with an electrical power or voltage source such as an external electrical outlet or an internal battery to activate LED driver 911, causing LED 960 to turn on.

In an "auto" position or setting, LED driver 911 is powered by the power source only in the event that sufficient electrical conductivity (e.g., a threshold level of conductivity, or sufficiently low resistance) exists between electrical conducting elements 907 in the neck 904. The required electrical conductivity, as needed for the "conditional completion" of the electrical circuit to power motorized device it, may be provided, for example, by an electrolyte solution containing ions (e.g., calcium, phosphate, fluoride, or peroxide ions) such as that generated from a combination of saliva, water, and toothpaste existing in the oral environment during use. When the electrical conductivity between conducting elements 907 is no longer present, the electrical circuit is incomplete, thereby deactivating LED driver 911 and LED 960. Thus, in an "auto" or automatic operating mode, LED driver 911 and LED 960 will not be activated when the toothbrush is stored since air is the medium between electrical conducting elements 907. According to some embodiments, when the brush is being rinsed outside the mouth, the water between electrical conducting elements 907 will not have sufficient electrical conductivity to activate LED driver 911 and LED 960.

According to some embodiments, it may be desired to require that the electrolyte solution (e.g., saliva or a water/saliva/toothpaste mixture), to which the toothbrush is exposed during use, have a threshold (or minimum) level of conductivity before LED driver 911 is activated. This threshold level of conductivity, for example, may be based on a threshold (or minimum) current needed to activate LED driver 911. This threshold conductivity, required to automatically turn on the toothbrush, may be associated with the electrical conductivity of saliva alone or an electrolyte solution having a relatively higher conductivity (e.g., an aqueous solution of toothpaste) or lower (e.g., a mixture of saliva and water) conductivity. For example, the threshold conductivity may be associated with a standard or model electrolyte solution designed to mimic the electrical conductivity of saliva having one or more specified, additional concentrations of dissolved ions such as calcium phosphate, fluoride, peroxide, and other ions or mixtures of ions.

In this manner, the automatic functioning of the oral care instrument can be made more or less sensitive to the particular conditions or conditions associated with the environment in which the instrument is used (i.e., the "use" condition(s) required to activate the instrument). It is also possible that the sensitivity of the instrument can be adjusted by, set by, or tailored to, the user (e.g., to avoid either activation of the instrument during "non-use" conditions or non-activation during "use" conditions) and thereby ensure effective functioning of the instrument in automatic mode.

In certain embodiments, the change in conductivity of the medium between electrical conducting elements 907 is measured by a sensing device 938, such as a circuit board 938 or other suitable sensing device, connected to electrical conducting elements 907 by electrical connections 940. In certain embodiments, sensing device 938 may measure the drop in resistance between conducting elements 907. When the conductivity change reaches a preset value as detected by sensing device 938, switch 932 may be activated so as to complete the electrical circuit to power LED driver 911. In such an embodiment, the electrical circuit need not include the electrolyte solution between conducting elements 907. That is, the electrolyte solution is used as a trigger to activate switch 932 by way of sensing device 938, but does not actually form part of the electrical circuit that powers LED driver 911.

In other embodiments, switch 932 could be activated based on the differential change in conductivity between conducting elements 907. It is to be appreciated that the level of electrolyte in the medium will vary from person to person, and/or may vary based on the formula of the oral care solution used. In such embodiments, these variations will not affect the current level delivered to the motor. Thus, for example, when using a sensitivity type toothpaste product having 5% KNO₃, the toothbrush would not operate differently than when used with a standard toothpaste product having a lower ionic strength.

According to other embodiments, when exposed to a solution with a threshold level of electrical conductivity, LED driver 911 and, therefore, the LED 960 itself may be set or adjusted (e.g., using a timer) to activate for a minimum duration. This ensures that the toothbrush or other instrument will function for at least enough time to effectively accomplish a given task (e.g., tooth cleaning and/or whitening). This also promotes continuous operation, even if contact between the instrument and the electrolyte solution is temporarily lost, for example, when a toothbrush is temporarily removed from the mouth during brushing. The minimum duration for activation of the LED (e.g., two minutes) may be fixed or may otherwise be set or adjusted according to a user's preferences.

As discussed above, the ability of a dental instrument to "activate" (e.g., to turn on a motor) when exposed to the environment in which it is used (e.g., an electrolyte solution in the mouth) can obviate the need for an "on/off switch or button, creating a simplified operation.

Another embodiment of a motorized device activated when conducting elements 907 are exposed to an electrolyte solution is shown in FIG. 71. A reservoir 944 is provided in handle 901 for storing an active agent. Conducting elements 907 are used to activate a pump 946, which causes a predetermined quantity of the active agent to be delivered from reservoir 944 through a channel 948 leading to a plurality of outlets 950 located in head 905. An exemplary delivery system for an active agent is described in US Application Ser. No. 11/457,086, the entire disclosure of which is incorporated herein. Other examples of oral care instruments that can activate an LED upon exposure of conducting elements 907 to an electrolyte solution will become readily apparent to those skilled in the art, given the benefit of this disclosure.

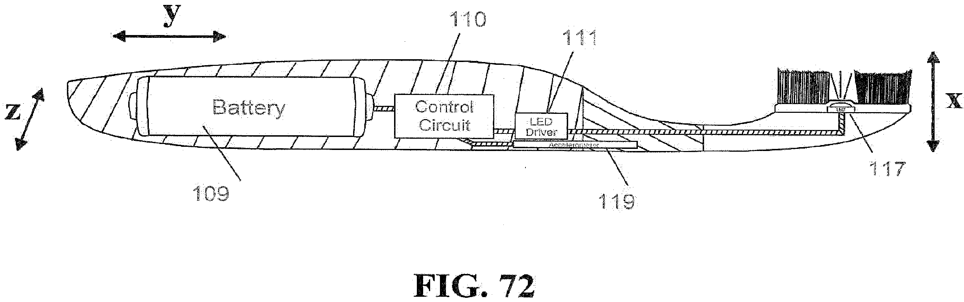

In yet another embodiment, the sensor may be one or more accelerometers within a dental hygiene implement that detect the user's brushing movement and compare it to previously stored brushing profiles to determine if the brush is in use or not. In the embodiment shown in Figure 72, the dental hygiene implement is a toothbrush 100 with an accelerometer 119 connected to a control circuit 110. If the accelerometer 119 detects that the brush is in use, the control circuit 110 will signal to the LED driver 111 to maintain the light source 117 until the brushing time has expired. The brushing time is adjustable, and may be set by either the manufacturer or the user. If the brushing time has expired, then the light source 117 will turn off and an indicator (not shown) will signal to the user that brushing has been completed. In one embodiment, the indicator is an additional LED light located on the handle of the toothbrush. In another embodiment, the indicator is an audio signal relayed to the user.

One or more accelerometers may be installed in the toothbrush to measure the x-, y- and z-axis of the toothbrush at any given moment while the user is brushing his teeth. The control circuit may signal to the LED driver to turn on the light source when the accelerometers indicate that the toothbrush is in an orientation within a predetermined range. In another embodiment of the invention, there is a time delay between the moment the accelerometers indicate that the toothbrush is in a brushing orientation and the moment where the control circuit signals to the LED driver to turn on the light source.

In yet another embodiment, the user may pre-program his brushing pattern to the control circuit such that the LED driver will only activate the light source only when the control circuit recognizes the user's brushing pattern. In one embodiment, the control circuit signals to the LED driver to activate the light source for a predetermined amount of time set by either the manufacturer or user. Alternatively, in another embodiment, the control circuit may continuously signal to the LED driver to keep the light source on until the accelerometer indicates that the user is no longer brushing.

In other embodiments of the invention, the sensor is a combination of two or more of these sensor types.

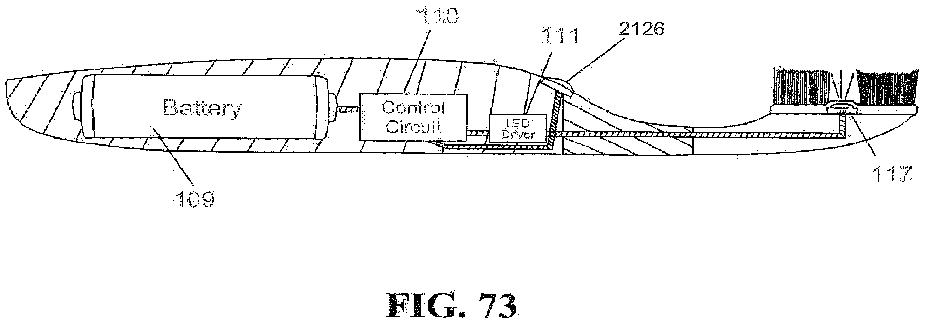

These embodiments and others may further include a fingerprint module mounted on the handle to verify if the user is authorized. In the embodiment shown in Figure 73, the dental hygiene implement is a toothbrush 100 having a LED 117 at the brush head connected to a LED driver 111 located in the handle of the toothbrush. The control circuit 110 is connected to the LED driver 111 as well as a battery 109 and a fingerprint module 2126. The fingerprint module 2126 may be initialized to recognize a user, with such information stored in the control circuit 110. Subsequent uses of the toothbrush will require the user to verify his identity by placing his fingerprint on the fingerprint module 2126.

In one embodiment, if the user's identity is correct, then the user may proceed to insert the brush head into his mouth and the LED will turn on in response to the installed sensor (not shown). If the user's identity is incorrect, then the LED will not turn on even if the installed sensor detects pressure, moisture, capacitance, etc. In another embodiment, the toothbrush 100 may further include an on/off switch where the fingerprint module and LED remain completely inactive in the "off setting. In yet another embodiment, the toothbrush 100 further includes an additional indicator to indicate whether the user is authorized. This indicator may be a visual light source that turns green when a user is authorized to use the brush. A red light source may be activated to indicate that the user is not authorized to use the toothbrush. Other types of indicators may be used, such as an audio indicator.

In another embodiment, the fingerprint module 2126 acts as the sensor for toothbrush 100. The LED will turn on once an authorized user places his fingerprint over the module. In this embodiment, it is intended for the user to place the fingerprint over the module only after the brush head is inserted within the mouth.

Certain modifications and improvements will occur to those skilled in the art upon reading the foregoing description. It should be understood that all such modifications and improvements have been omitted for the sake of conciseness and readability, but are properly within the scope of the following claims.

## Claims

1. A toothbrush (100, 130, 160) having a light source comprising:
a brush including a battery (109), a source of light (117, 131) with a wavelength in the range of 400nm to 500nm, electronics (110, 111) coupled to the battery and the source of light to supply electricity from the battery to the source at current and voltage that is adapted to cause the source to emit a light, the brush including a handle (114, 153) and a distal end (118, 134);
the distal end including bristles (113, 132) mounted on the distal end and the source of light, the distal end being equipped with a first electrical contact (116, 133, 152, 162) accessible on a first exterior surface, **characterised by** at least a portion of the handle being equipped with a second electrical contact (115, 151, 161) accessible on a second exterior surface, the first and the second electrical contacts being connected to the electronics, said electronics being adapted to determine whenever the distal end is outside the environment of the mouth, and permit activation of the source of light only when the source of light is inside the mouth of the user and a current loop is completed through the first and second electrical contacts and the body of the user.

2. A toothbrush (100, 130, 160) as claimed in claim 1 wherein the source of light (117, 131) emits light in the range about 400nm to about 420nm.

3. A toothbrush (100, 130, 160) as claimed in claim 1 wherein the electronics (110) enables the current loop, and current in the loop is sensed by the electronics at a detection current threshold at a sub-microampere level at around one volt of potential difference.

4. A toothbrush as claimed in claim 1 wherein the distal end includes a brush head (157) with bristles (156) that is replaceably mounted on the handle (146) and has a conductor (172) to connect to a mating conductor (170) in the brush handle to allow sensor current to flow across the contacting conductors (170, 172).

5. A toothbrush as claimed in claim 1 wherein the distal end includes a brush head (157) with the bristles (156) that is replaceably mounted on the handle (146), and the brush head and handle have conductive plastic parts that are in contact when the brush head is mounted on the handle to allow sensor current to flow across the contacting conducting plastic parts.

6. A toothbrush (100, 130, 160) as claimed in claim 1 wherein the electronics (110) includes an ultralow power comparator using CMOS electronics.

7. A toothbrush (100, 130, 160) as claimed in claim 1 wherein the electronics (110) includes a timer coupled to a light or noise maker to activate the light or noise maker after a predetermined time interval, so a user knows sufficient brushing has occurred.

8. A toothbrush (100, 130, 160) as claimed in claim 1 wherein the electronics (110) includes a make/break switch on the brush and accessible to the user, to enable the user to prevent operation by breaking a current path through the switch and to enable operation by making a current path through the switch.

9. A toothbrush (100, 130, 160) as claimed in claim 1 wherein the toothbrush is a power toothbrush.

10. A toothbrush (100) as claimed in claim 1 further including a fingerprint detection module (2126) mounted on the handle to verify if the user is authorized.

11. The toothbrush of claim 1, wherein the electronics further includes an AC signal loop sensor.

## Patentansprüche

1. Zahnbürste (100, 130, 160), die eine Lichtquelle hat, umfassend:
eine Bürste, aufweisend eine Batterie (109), eine Quelle von Licht (117, 131) mit einer Wellenlänge in dem Bereich von 400 nm bis 500 nm, Elektronik (110, 111), die mit der Batterie und der Quelle von Licht gekoppelt ist, um Elektrizität von der Batterie mit einer Stromstärke und einer Spannung an die Quelle zu liefern, die dazu angepasst sind, die Quelle dazu zu veranlassen, Licht abzugeben, wobei die Bürste einen Handgriff (114, 153) und ein distales Ende (118, 134) aufweist;
wobei das distale Ende Borsten (113, 132) aufweist, die auf dem distalen Ende und der Quelle von Licht angebracht sind, wobei das distale Ende mit einem ersten elektrischen Kontakt (116, 133, 152, 162) ausgestattet ist, der auf einer ersten Außenoberfläche zugänglich ist, **dadurch gekennzeichnet, dass** mindestens ein Teil des Handgriffs mit einem zweiten elektrischen Kontakt (115, 151, 161) ausgestattet ist, der auf einer zweiten Außenoberfläche zugänglich ist, wobei der erste und der zweite elektrische Kontakt mit der Elektronik verbunden sind, wobei die Elektronik dazu angepasst ist festzustellen, wann das distale Ende außerhalb der Umgebung des Munds ist, und eine Aktivierung der Quelle von Licht nur dann erlaubt, wenn die Quelle von Licht innerhalb des Munds des Benutzers ist und über den ersten und den zweiten elektrischen Kontakt und den Körper des Benutzers ein Stromkreis geschlossen ist.

2. Zahnbürste (100, 130, 160) gemäß Anspruch 1, wobei die Quelle von Licht (117, 131) Licht in dem Bereich von ungefähr 400 nm bis ungefähr 420 nm abgibt.

3. Zahnbürste (100, 130, 160) gemäß Anspruch 1, wobei die Elektronik (110) den Stromkreis freischaltet und Strom in dem Stromkreis von der Elektronik bei einem Erfassungs-Strom-Schwellenwert auf einem Sub-Mikroampere-Niveau bei ungefähr einem Volt Potentialdifferenz erfasst wird.

4. Zahnbürste gemäß Anspruch 1, wobei das distale Ende einen Bürstenkopf (157) mit Borsten (156) aufweist, der austauschbar auf dem Handgriff (146) angebracht ist und einen Leiter (172) hat, um mit einem zusammenpassenden Leiter (170) in dem Bürstenhandgriff verbunden zu werden, um es einem Sensorstrom zu ermöglichen, über die in Kontakt stehenden Leiter (170, 172) zufließen.

5. Zahnbürste gemäß Anspruch 1, wobei das distale Ende einen Bürstenkopf (157) mit den Borsten (156) aufweist, der austauschbar auf dem Handgriff (146) angebracht ist, und der Bürstenkopf und der Handgriff leitfähige Kunststoffteile aufweisen, die in Kontakt sind, wenn der Bürstenkopf auf dem Handgriff angebracht ist, um es Sensorstrom zu ermöglichen, über die in Kontakt stehenden Kunststoffteile zu fließen.

6. Zahnbürste (100, 130, 160) gemäß Anspruch 1, wobei die Elektronik (110) einen Ultra-Niederspannungs-Komparator aufweist, der CMOS-Elektronik verwendet.

7. Zahnbürste (100, 130, 160) gemäß Anspruch 1, wobei die Elektronik (110) einen Timer aufweist, der mit einer Licht- oder Geräusch-Erzeugungseinrichtung gekoppelt ist, um die Licht- oder Geräusch-Erzeugungseinrichtung nach einem vorbestimmten Zeitintervall zu aktivieren, sodass ein Benutzer weiß, dass ein ausreichendes Bürsten stattgefunden hat.

8. Zahnbürste (100, 130, 160) gemäß Anspruch 1, wobei die Elektronik (110) einen Ein-Aus-Schalter auf der Bürste und für den Benutzer zugänglich aufweist, um es dem Benutzer zu ermöglichen, einen Betrieb zu verhindern, indem er einen Strompfad über den Schalter unterbricht, und einen Betrieb zu ermöglichen, indem er einen Strompfad über den Schalter herstellt.

9. Zahnbürste (100, 130, 160) gemäß Anspruch 1, wobei die Zahnbürste eine elektrische Zahnbürste ist.

10. Zahnbürste (100) gemäß Anspruch 1, ferner aufweisend ein Fingerabdruck-Erkennungsmodul (2126), das auf dem Handgriff angebracht ist, um zu überprüfen, ob der Benutzer autorisiert ist.

11. Zahnbürste gemäß Anspruch 1, wobei die Elektronik ferner einen Wechselspannungssignal-Loop-Sensor aufweist.

## Revendications

1. Brosse à dents (100, 130, 160) comportant une source de lumière comprenant :
une brosse comprenant une batterie (109), une source de lumière (117, 131) avec une longueur d'onde dans la plage de 400 nm à 500 nm, une électronique (110, 111) couplée à la batterie et à la source de lumière pour fournir de l'électricité de la batterie à la source à un courant et une tension qui sont adaptés pour amener la source à émettre une lumière, la brosse comprenant un manche (114, 153) et une extrémité distale (118, 134) ;
l'extrémité distale comprenant des poils (113, 132) montés sur l'extrémité distale et la source de lumière, l'extrémité distale étant équipée d'un premier contact électrique (116, 133, 152, 162) accessible sur une première surface extérieure, **caractérisée en ce qu'**au moins une partie du manche est équipée d'un deuxième contact électrique (115, 151, 161) accessible sur une deuxième surface extérieure, les premier et deuxième contacts électriques étant connectés à l'électronique, ladite électronique étant conçue pour déterminer quand l'extrémité distale est à l'extérieur de l'environnement de la bouche, et permettre l'activation de la source de lumière uniquement lorsque la source de lumière est à l'intérieur de la bouche de l'utilisateur et qu'une boucle de courant est réalisée par les premier et deuxième contacts électriques et le corps de l'utilisateur.

2. Brosse à dents (100, 130, 160) selon la revendication 1, dans laquelle la source de lumière (117, 131) émet une lumière dans la plage d'environ 400 nm à environ 420 nm.

3. Brosse à dents (100, 130, 160) selon la revendication 1, dans laquelle l'électronique (110) active la boucle de courant, et le courant dans la boucle est détecté par l'électronique à un seuil de courant de détection au niveau des sous-microampères à une différence de potentiel d'environ un volt.

4. Brosse à dents selon la revendication 1, dans laquelle l'extrémité distale comprend une tête de brosse (157) avec des poils (156) qui est montée de manière remplaçable sur le manche (146) et comporte un conducteur (172) pour une connexion à un conducteur correspondant (170) dans le manche de brosse pour permettre la circulation d'un courant de capteur à travers les conducteurs de contact (170, 172).

5. Brosse à dents selon la revendication 1, dans laquelle l'extrémité distale comprend une tête de brosse (157) avec les poils (156) qui est montée de manière remplaçable sur le manche (146), et la tête de brosse et le manche comportent des parties en matière plastique conductrices qui sont en contact lorsque la tête de brosse est montée sur le manche pour permettre la circulation d'un courant de capteur à travers les parties en matière plastique conductrices en contact.

6. Brosse à dents (100, 130, 160) selon la revendication 1, dans laquelle l'électronique (110) comprend un comparateur de puissance ultra faible utilisant une électronique CMOS.

7. Brosse à dents (100, 130, 160) selon la revendication 1, dans laquelle l'électronique (110) comprend un registre d'horloge couplé à un dispositif de production de lumière ou de bruit pour activer le dispositif de production de lumière ou de bruit après un intervalle de temps prédéterminé, ainsi un utilisateur sait qu'un brossage suffisant a été effectué.

8. Brosse à dents (100, 130, 160) selon la revendication 1, dans laquelle l'électronique (110) comprend un commutateur de production/interruption sur la brosse et accessible à l'utilisateur, pour permettre à l'utilisateur d'empêcher le fonctionnement en interrompant un trajet de courant à travers le commutateur et pour permettre le fonctionnement en réalisant un trajet de courant à travers le commutateur.

9. Brosse à dents (100, 130, 160) selon la revendication 1, dans laquelle la brosse à dents est une brosse à dents électrique.

10. Brosse à dents (100) selon la revendication 1 comprenant en outre un module de détection d'empreintes digitales (2126) monté sur le manche pour vérifier si l'utilisateur est autorisé.

11. Brosse à dents selon la revendication 1, dans laquelle l'électronique comprend en outre un capteur de boucle de signal alternatif.
